# EUROPEAN PATENT APPLICATION

(11) **EP 2 525 224 A1**
(43) Date of publication of application: **21.11.2012**
(21) Application number: 11166799.4
(22) Date of filing: 19.05.2011
(51) Int. Cl.: G01N 33/50, G01N 33/574

(54) **VDAC1-S as a cell marker**

(71) Applicant: Centre National de la Recherche Scientifique, 75016 Paris Cedex 16 (FR); Université Nice Sophia Antipolis, 06108 Nice Cedex 2 (FR)
(72) Inventor: Mazure, Nathalie, 06690 Tourette-Levens (FR)
(74) Representative: Le Coupanec, Pascale A.M.P.

(57) **Abstract**

The instant invention relates to the use of C-terminally truncated form of VDAC1 as a cell marker of apoptosis-resistance phenotype of hypoxic cancer cells.

SEQUENCE LISTING

<110>  Centre National de la Recherche Scientifique (CNRS)
       Université Nice Sophia Antipolis

<120>  VDAC1-S as a cell marker

<130>  PR92989 KLP/HGC

<160>  22

<170>  PatentIn version 3.5

<210>  1
<211>  852
<212>  DNA
<213>  Homo sapiens

<400>  1
atggctgtgc cacccacgta tgccgatctt ggcaaatctg ccagggatgt cttcaccaag      60

ggctatggat ttggcttaat aaagcttgat ttgaaaacaa aatctgagaa tggattggaa     120

tttacaagct caggctcagc caacactgag accaccaaag tgacgggcag tctggaaacc     180

aagtacagat ggactgagta cggcctgacg tttacagaga aatggaatac cgacaataca     240

ctaggcaccg agattactgt ggaagatcag cttgcacgtg gactgaagct gaccttcgat     300

tcatccttct cacctaacac tgggaaaaaa aatgctaaaa tcaagacagg gtacaagcgg     360

gagcacatta acctgggctg cgacatggat ttcgacattg ctgggccttc catccggggt     420

gctctggtgc taggttacga gggctggctg gccggctacc agatgaattt tgagactgca     480

aaatcccgag tgacccagag caactttgca gttggctaca agactgatga attccagctt     540

cacactaatg tgaatgacgg gacagagttt ggcggctcca tttaccagaa agtgaacaag     600

aagttggaga ccgctgtcaa tcttgcctgg acagcaggaa acagtaacac gcgcttcgga     660

atagcagcca agtatcagat tgaccctgac gcctgcttct cggctaaagt gaacaactcc     720

agcctgatag gtttaggata cactcagact ctaaagccag gtattaaact gacactgtca     780

gctcttctgg atggcaagaa cgtcaatgct ggtggccaca agcttggtct aggactggaa     840

tttcaagcat aa                                                         852

<210>  2
<211>  717
<212>  DNA
<213>  Homo sapiens

<400>  2
atggctgtgc cacccacgta tgccgatctt ggcaaatctg ccagggatgt cttcaccaag      60

ggctatggat ttggcttaat aaagcttgat ttgaaaacaa aatctgagaa tggattggaa     120

tttacaagct caggctcagc caacactgag accaccaaag tgacgggcag tctggaaacc     180

aagtacagat ggactgagta cggcctgacg tttacagaga aatggaatac cgacaataca     240

ctaggcaccg agattactgt ggaagatcag cttgcacgtg gactgaagct gaccttcgat     300

tcatccttct cacctaacac tgggaaaaaa aatgctaaaa tcaagacagg gtacaagcgg     360

gagcacatta acctgggctg cgacatggat ttcgacattg ctgggccttc catccggggt     420

gctctggtgc taggttacga gggctggctg gccggctacc agatgaattt tgagactgca 480

aaatcccgag tgacccagag caactttgca gttggctaca agactgatga attccagctt 540

cacactaatg tgaatgacgg gacagagttt ggcggctcca tttaccagaa agtgaacaag 600

aagttggaga ccgctgtcaa tcttgcctgg acagcaggaa acagtaacac gcgcttcgga 660

atagcagcca agtatcagat tgaccctgac gcctgcttct cggctaaagt gaacaac 717

<210> 3
<211> 283
<212> PRT
<213> Homo sapiens

<400> 3

Met Ala Val Pro Pro Thr Tyr Ala Asp Leu Gly Lys Ser Ala Arg Asp
1               5                   10                  15

Val Phe Thr Lys Gly Tyr Gly Phe Gly Leu Ile Lys Leu Asp Leu Lys
            20                  25                  30

Thr Lys Ser Glu Asn Gly Leu Glu Phe Thr Ser Ser Gly Ser Ala Asn
        35                  40                  45

Thr Glu Thr Thr Lys Val Thr Gly Ser Leu Glu Thr Lys Tyr Arg Trp
    50                  55                  60

Thr Glu Tyr Gly Leu Thr Phe Thr Glu Lys Trp Asn Thr Asp Asn Thr
65                  70                  75                  80

Leu Gly Thr Glu Ile Thr Val Glu Asp Gln Leu Ala Arg Gly Leu Lys
                85                  90                  95

Leu Thr Phe Asp Ser Ser Phe Ser Pro Asn Thr Gly Lys Lys Asn Ala
            100                 105                 110

Lys Ile Lys Thr Gly Tyr Lys Arg Glu His Ile Asn Leu Gly Cys Asp
        115                 120                 125

Met Asp Phe Asp Ile Ala Gly Pro Ser Ile Arg Gly Ala Leu Val Leu
    130                 135                 140

Gly Tyr Glu Gly Trp Leu Ala Gly Tyr Gln Met Asn Phe Glu Thr Ala
145                 150                 155                 160

Lys Ser Arg Val Thr Gln Ser Asn Phe Ala Val Gly Tyr Lys Thr Asp
                165                 170                 175

Glu Phe Gln Leu His Thr Asn Val Asn Asp Gly Thr Glu Phe Gly Gly
            180                 185                 190

Ser Ile Tyr Gln Lys Val Asn Lys Lys Leu Glu Thr Ala Val Asn Leu
        195                 200                 205

Ala Trp Thr Ala Gly Asn Ser Asn Thr Arg Phe Gly Ile Ala Ala Lys
    210                 215                 220

Tyr Gln Ile Asp Pro Asp Ala Cys Phe Ser Ala Lys Val Asn Asn Ser
225                 230                 235                 240

Ser Leu Ile Gly Leu Gly Tyr Thr Gln Thr Leu Lys Pro Gly Ile Lys
                245                 250                 255

Leu Thr Leu Ser Ala Leu Leu Asp Gly Lys Asn Val Asn Ala Gly Gly
            260                 265                 270

His Lys Leu Gly Leu Gly Leu Glu Phe Gln Ala
        275                 280

<210>  4
<211>  239
<212>  PRT
<213>  Homo sapiens

<400>  4

Met Ala Val Pro Pro Thr Tyr Ala Asp Leu Gly Lys Ser Ala Arg Asp
1               5                   10                  15

Val Phe Thr Lys Gly Tyr Gly Phe Gly Leu Ile Lys Leu Asp Leu Lys
            20                  25                  30

Thr Lys Ser Glu Asn Gly Leu Glu Phe Thr Ser Ser Gly Ser Ala Asn
        35                  40                  45

Thr Glu Thr Thr Lys Val Thr Gly Ser Leu Glu Thr Lys Tyr Arg Trp
    50                  55                  60

Thr Glu Tyr Gly Leu Thr Phe Thr Glu Lys Trp Asn Thr Asp Asn Thr
65                  70                  75                  80

Leu Gly Thr Glu Ile Thr Val Glu Asp Gln Leu Ala Arg Gly Leu Lys
                85                  90                  95

Leu Thr Phe Asp Ser Ser Phe Ser Pro Asn Thr Gly Lys Lys Asn Ala
            100                 105                 110

Lys Ile Lys Thr Gly Tyr Lys Arg Glu His Ile Asn Leu Gly Cys Asp
        115                 120                 125

Met Asp Phe Asp Ile Ala Gly Pro Ser Ile Arg Gly Ala Leu Val Leu
    130                 135                 140

Gly Tyr Glu Gly Trp Leu Ala Gly Tyr Gln Met Asn Phe Glu Thr Ala
145                 150                 155                 160

Lys Ser Arg Val Thr Gln Ser Asn Phe Ala Val Gly Tyr Lys Thr Asp
                165                 170                 175

Glu Phe Gln Leu His Thr Asn Val Asn Asp Gly Thr Glu Phe Gly Gly
            180                 185                 190

Ser Ile Tyr Gln Lys Val Asn Lys Lys Leu Glu Thr Ala Val Asn Leu
        195                 200                 205

Ala Trp Thr Ala Gly Asn Ser Asn Thr Arg Phe Gly Ile Ala Ala Lys
    210                 215                 220

Tyr Gln Ile Asp Pro Asp Ala Cys Phe Ser Ala Lys Val Asn Asn
225                 230                 235

<210>  5
<211>  44
<212>  PRT
<213>  Homo sapiens

<400>  5

Ser Ser Leu Ile Gly Leu Gly Tyr Thr Gln Thr Leu Lys Pro Gly Ile
1               5                   10                  15

Lys Leu Thr Leu Ser Ala Leu Leu Asp Gly Lys Asn Val Asn Ala Gly
            20                  25                  30

Gly His Lys Leu Gly Leu Gly Leu Glu Phe Gln Ala
        35                  40

<210>  6
<211>  19
<212>  DNA
<213>  Artificial sequence

<220>
<223>  siRNA

<400>  6
gauacacuca gacucuaaa                                                    19

<210>  7
<211>  20
<212>  DNA
<213>  Artificial sequence

<220>
<223>  Primer

<400>  7
gccgctcgct cggctccgct                                                   20

<210>  8
<211>  19
<212>  DNA
<213>  Artificial sequence

<220>

<223>  Primer

<400>  8
gcattgaagg ggaggatct                                                    19


<210>  9
<211>  18
<212>  DNA
<213>  Artificial sequence

<220>
<223>  Primer

<400>  9
gttggtgaaa ctggatgt                                                     18


<210>  10
<211>  21
<212>  DNA
<213>  Artificial sequence

<220>
<223>  Primer

<400>  10
atggcgaccc acggacagac t                                                 21


<210>  11
<211>  18
<212>  DNA
<213>  Artificial sequence

<220>
<223>  Primer

<400>  11
tcctgtagcc cactgcaa                                                     18


<210>  12
<211>  20
<212>  DNA
<213>  Artificial sequence

<220>
<223>  Primer

<400>  12
gttgccctgg cggacagaga                                                   20


<210>  13
<211>  21
<212>  DNA
<213>  Artificial sequence

<220>
<223>  Primer

<400>  13
ccaagagatt tctgtcccta c                                                 21


<210>  14
<211>  21
<212>  DNA

<213>  Artificial sequence

<220>
<223>  Primer

<400>  14
atgtgtaaca caccaacgta c                                                21


<210>  15
<211>  29
<212>  DNA
<213>  Artificial sequence

<220>
<223>  Primer

<400>  15
ctctagatca aatcccaagc caaccttgt                                        29


<210>  16
<211>  19
<212>  DNA
<213>  Artificial sequence

<220>
<223>  siRNA

<400>  16
gaagauuugg ccuuaauau                                                   19


<210>  17
<211>  21
<212>  DNA
<213>  Artificial sequence

<220>
<223>  siRNA

<400>  17
gucuguaacu auggacuuat t                                                21


<210>  18
<211>  21
<212>  DNA
<213>  Artificial sequence

<220>
<223>  siRNA

<400>  18
ccaaacuguc acagaauaat t                                                21


<210>  19
<211>  20
<212>  DNA
<213>  Artificial sequence

<220>
<223>  siRNA

<400>  19
cagagguucg agaaaagatt                                                  20

<210>  20
<211>  21
<212>  DNA
<213>  Artificial sequence

<220>
<223>  siRNA

<400>  20
ggaugaaggu agaaauggat t                                                21


<210>  21
<211>  30
<212>  DNA
<213>  Artificial sequence

<220>
<223>  Primer

<400>  21
gaaagcttaa tggctgtgcc acccacgtat                                       30


<210>  22
<211>  30
<212>  DNA
<213>  Artificial sequence

<220>
<223>  Primer

<400>  22
atgaattctt taggagttgt tcactttagc                                       30

## Description

### {FIELD}

The instant invention relates to cell markers for cancer.

More specifically, the invention relates to cell markers, or biomarkers, of apoptosis-resistance phenotype of hypoxic cancer cells and their uses as targets in cancer diagnosis and prognosis of cancer treatment. More particularly, the present invention relates to methods for evaluating the apoptosis-resistance phenotype of hypoxic cancer cells, for assessing responsiveness of an individual suffering from a cancer to a cancer treatment, and to stage cancer. The instant invention also relates to methods for screening candidate active agents useful for restoring apoptosis sensitivity into hypoxic cancer cells and for cancer treatment.

### {BACKGROUND}

A cancer is a class of diseases in which a cell or a group of cells display uncontrolled growth invasion that intrudes upon and destroys adjacent tissues and sometimes metastasis to other locations in the body.

The resistance of cancer cells to chemotherapy has been widely attributed to the hypoxic nature of tumours (Bertout et al., Nat Rev Cancer, 2008, 8:967). Because of the poor vascularization and oxygenation of the internal parts of growing tumours, the ATP production in tumour cells relies upon glycolytic metabolism instead of essentially mitochondrial oxidative respiration. The mechanisms behind this switch in metabolism implicate oncogenic activation, in particular Myc, and the transcription factor hypoxia-inducible factor (HIF), which under limiting oxygen conditions, promotes glycolysis by up-regulating glycolytic enzymes and restricts mitochondrial respiration by up regulating pyruvate dehydrogenase kinase 1, an inhibitor of pyruvate dehydrogenase that drives pyruvate toward mitochondrial oxidative phosphorylation. Thus, hypoxia promotes cell survival through maintenance of ATP production and blocks production of cytotoxic reactive oxygen species formed during mitochondrial oxidative phosphorylation.

Also, mitochondria is known to play an important role in the programmed cell death processes or apoptosis in particular through the release of cytochrome c (cyto. c), from the inter membrane space of mitochondria to the cytoplasm.

Although controversial, two major models have been described to explain the release of cyto. c, which activates caspases and induces apoptosis (Kroemer, 2007, Physiol Rev 87, 99-163). One model proposes the formation of pores in the OMM that are made up of the pro-apoptotic proteins Bid, Bax and Bak and anti-apoptotic proteins Bcl-X_{L} and Bcl-2. A second model proposes disruption of the OMM due to the formation/opening of a mitochondrial megapore called the permeability transition pore complex (PTPC). The PTPC has been proposed to be composed of the voltage-dependent anion channel (VDAC) in the OMM, the adenine nucleotide translocator in the inner mitochondrial membrane (IMM) and cyclophilin D in the mitochondrial matrix (Galluzzi, 2007, Nat Cell Biol, 9, 487-489; Shoshan-Barmatz, 2010, Curr Pharm Des, 12, 2249-2270; Kroemer, 2008, Cancer Cell 13, 472-482). VDAC in particular controls both cell survival and death by regulating mitochondrial import and export of metabolites including ATP, and by interacting with anti-apoptotic proteins such as Bcl-2 and hexokinase.

The understanding of how hypoxic cancer cells become resistant to chemotherapy and how to distinguish apoptosis resistant cancer cells from non-resistant cells appears to be essential for improving and combating cancer. Yet, the mechanisms behind chemotherapy resistance in hypoxic tumours are largely unknown.

Therefore, there is a need for cell markers, or biomarkers, useful for characterizing the apoptosis-resistance phenotype of hypoxic cancer cells.

There is a need for cell marker, or biomarkers, for staging a cancer, or assessing responsiveness of an individual suffering from a cancer to a cancer treatment.

There is also a need for a simple and reliable method for identifying apoptosis-resistant hypoxic cancer cells.

There is also a need for treatments allowing preventing, reducing or suppressing the resistance of cancer cells to apoptosis-inducing agents.

There is also a need for the identification of novel anticancer active agents to which cancer cells and tumours do not become resistant.

There is also a need for a method allowing assessing responsiveness of a individual suffering from a cancer to a cancer treatment.

The present invention has for object to meet these needs.

### {SUMMARY}

According to one of its first aspects, the instant invention relates to an isolated peptide wherein:
- it has a molecular weight of about 5 kDa less than a naturally expressed long isoform of VDAC1, in polyacrylamide gel electrophoresis migration, and
- it is recognized by an antibody able to specifically bind to an amino acids sequence corresponding to amino acids residues 150-250 of the amino acids sequence as set forth in SEQ ID NO: 3, but not by an antibody able to bind to an amino acids sequence corresponding to amino acids residues 234 to 283 of the amino acids sequence SEQ ID NO: 3

As examples of useful first and second antibodies suitable for the invention, one may mention the antibody ab15895 and ab28777 from ABCAM.

According to another aspect, the instant invention relates to an isolated peptide represented by an amino acids sequence consisting in 230 to 250 contiguous N-terminal amino acids of an amino acids sequence as set forth by SEQ ID NO: 3, or an analog thereof having at least an identity of sequence of at least 90% and a similar biology activity.

Surprisingly, the inventors have observed that the expression of a short isoform, C-terminally truncated, of the voltage-dependent anion channel 1, VDAC1 (VDAC1-S), was induced into hypoxic cancer cells and correlated with the change of morphology of mitochondria in a limiting oxygen microenvironment or hypoxia and with the apoptosis-resistance phenotype of cancer cells.

In the specification, the term "VDAC1-S" is used to designate the peptides of the invention, as defined above and hereafter. In the specification, the term "VDAC1-L" is used to designate the long isoform of VDAC1 naturally expressed, and except otherwise stated "VDAC1" is used to designate VDAC1-S and VDAC1-L, or total VDAC-1. VDAC1-L may be represented by SEQ ID NO: 3 or P21796 (Ref. PUBMED), or analog, or variant, thereof having at least an identity of sequence of at least 90% and a similar biology activity.

The hypoxic cancer cells were observed to be resistant to the staurosporine or etoposide induced-apoptosis, whereas the prevention or the inhibition of the expression of VDAC1-S was observed to restore the sensitivity of hypoxic cancerous cells to theses apoptotic agents. It has also been observed that VDAC1-S has the same channel activity and voltage-dependancy as the long isoform of VDAC1 (VDAC1-L), and that when an artificially constructed C-terminally truncated form of VDAC1-L, having an amino acid sequence as set forth in SEQ ID NO: 4 was expressed in normoxic cells, a resistance to staurosporine-induced apoptosis was obtained.

According to another object, the invention relates to the use of a peptide VDAC1-S as a cell marker of apoptosis-resistance phenotype of hypoxic cancer cells.

According to another aspect, the invention relates to the use of a peptide VDAC1-S as a biomarker for staging a cancer.

According to another object, the invention relates to the use of a peptide VDAC1-S as a biomarker for assessing responsiveness of an individual suffering from a cancer to a cancer treatment.

According to an embodiment, a use of the invention comprises a determination of the level of expression or activity of a peptide of the invention.

According to another embodiment, a ratio of a level of expression or activity of a peptide of the invention to a level of expression or activity of total VDAC1 is determined. Therefore, the invention relates also to a ratio VDAC1-S/VDAC1 as cell marker or biomarker in the uses and methods of the invention.

According to an embodiment, a presence or an increased level of expression and/or activity of a peptide of the invention, or of a ratio of the invention, is indicative of an apoptosis-resistance phenotype, or of an advanced stage of a cancer, or of a low-responsiveness to a cancer treatment.

According to another embodiment, a use of VDAC1-S according to the invention, and in particular an observed presence or increased level of expression and/or activity of VDAC1-S may be associated with a presence of mitochondria of enlarged phenotype.

In accordance with the invention, the expression "cell marker" or "biomarker" is intended to refer to a constituent of a cell or a tissue which may be determined directly or indirectly, for example by direct visualisation or after isolation and labeling with a fluorescent or an isotopic tag, or else by a dosage method, and which is indicative of a physiological, biochemical or morphological state of said cell or tissue. Accordingly, a cell marker or biomarker may be used to qualify a physiological, biochemical or morphological state of an individual in need thereof or of a biological tissue of said individual.

Within the invention, the term "phenotype" is intended to refer to the morphological, biochemical and physiological characteristics of a tissue, a cell or a cell organelle.

In accordance with the invention, the expression "apoptosis-resistance phenotype" is intended to refer to a phenotype of a cell or a tissue which is or becomes resistant to at least one agent, chemical or physical, known to cause apoptosis.

Within the invention, the term "hypoxic" intends to qualify a condition where the oxygen is depleted or is in a subnormal concentration. For example, hypoxic cell culture conditions may be obtained by subjecting cultured cells to an atmosphere of 1% O₂, 94% nitrogen and 5% CO₂ for at least 48 hours. "Hypoxia" intends to refer to a reduction in the normal concentration of tissue oxygen.

Within the invention, a "cancer treatment" intends to refer to a treatment presented to stabilize, reduce, suppress, prevent and/or cure a cancer disease, and to cause apoptosis to cancer cells.

Within the invention, the expression "low-responsiveness to a cancer treatment" is intended to qualify an inefficient level of pharmacological effect observed in an individual suffering from a cancer to whom is administered a cancer treatment presumed to be effective to the type of cancer affecting the individual. In a low-responsiveness individual, although receiving the presumed appropriate treatment, the cancer is not stabilized, reduced, or cured, but instead, the cancer tends to progress. By contrast, a responsive individual suffering from a cancer to who is administered a cancer treatment presumed to be effective to the type of cancer affecting the individual, the cancer tends to be stabilized, reduced or cured.

The assessment of stabilization, reduction, curing or progression of a cancer belongs to the field of the skilled artisan in the art.

According to another of its aspects, the present invention relates to a method for determining an apoptosis-resistance phenotype of at least one isolated hypoxic cancer cell suspected to be apoptosis-resistant comprising at least the steps of:
a) determining in said hypoxic cancer cell a presence or an absence, or a level of expression and/or activity of a peptide according to the invention, and
b) comparing said determined presence or absence, or level of expression and/or activity to a control value or range.

According to another of its aspects, the present invention relates to a method for assessing responsiveness of an individual suffering from a cancer to a cancer treatment, the method comprising at least the steps of:
a) determining a presence or an absence, or a level of expression and/or activity of a peptide according to the invention in a first biological sample obtained from the individual before the treatment has been administered to said individual,
b) determining a presence or an absence, or a level of expression and/or activity of a peptide according to the invention in a second biological sample obtained from the individual after the treatment has been administered to said individual, and
c) comparing the determination obtained from the first biological to the determination obtained from the second biological sample.

According to another of its aspects, the present invention relates to a method for staging a cancer in an individual having a cancer, the method comprising at least the steps of:
a) determining in a sample taken from a cancerous tissue of said individual a level of expression and/or activity of a peptide according to the invention, and
b) comparing said level of expression or activity to a control value or range.

Within the invention, the term "to determine" is intended to refer to a qualitative and/or a quantitative detection.

The methods and uses of the invention, instead of determining a level of expression and/or activity of a peptide of the invention, one may determine a ratio VDAC1-S/VDAC-1 as above-defined.

According to one embodiment, a method of the invention may, after the step of determining a level of expression or activity of a peptide of the invention, further comprise steps of determining a level of expression or activity of total VDAC1, and of determining a ratio of said level of expression or activity of said peptide to said level of expression or activity of total VDAC1. In such embodiment, the step of comparing is performed with said ratio.

Within the invention, the expression "control value or range" intends to refer to a value or a range of values of a parameter determined under basal conditions.

For example, with respect to the level of expression of VDAC1-S, the control value or range may be a value or range of values corresponding to a level of expression of VDAC1-S in a cancer cell maintained in normoxia when influence of oxygen concentration is assayed. Alternatively, it may be a level of expression of VDAC1-S in a hypoxic apoptosis-resistant cancer cell without a candidate active anticancer agent when said candidate active anticancer agent is to be assayed.

Typically, for a method of diagnosing, a control value or range may be obtained by determining VDAC1-S in a set of individuals not having a cancer, or in a healthy tissue, or presumed healthy tissue, from an individual suffering from a cancer. Preferably the control is matched to the patient with respect to some attribute(s) (e.g. age). Depending upon control value or range and the difference between the determined and control value or range, the patient can be diagnosed as having or not apoptosis-resistant cancer cells or a cancer resistant or not to a cancer treatment.

Measurement of the control value or range need not be made contemporaneously, it may be a historical measurement.

As above-indicated, the step of comparing may be carried out with a ratio of the level of expression or activity of a peptide of the invention to the level of expression or activity of total VDAC1, i.e. including VADC1-S and VDAC1-L, VDAC1-S/VDAC1. In such case, the control value or range for a ratio VDAC1-S/VDAC1 may be determined and obtained according to similar strategies than the ones above-exposed for the control value or range for VDAC1-S.

According to another aspect, the instant invention relates to a method for screening candidate active agents able to restore apoptosis-sensitivity to an apoptosis-resistant hypoxic cancer cell comprising at least the steps of:
a) providing at least one isolated cell able to express a naturally expressed long isoform of VDAC1 or a peptide according to anyone of claims 1 to 4 or a non-human transgenic animal able to express a naturally expressed long isoform of VDAC1 or said peptide in conditions suitable for the expression of said naturally expressed long isoform of VDAC1 or said peptide,
b) contacting said isolated cell or said non-human transgenic animal with at least one candidate active agent to be screened,
c) determining in said isolated cell or in said non-human transgenic animal a presence or absence, or a level of expression and/or activity of said naturally expressed long isoform of VDAC1 or said peptide, and
d) comparing said determined presence or absence, or level of expression and/or activity to a control value or range.

According to another of its aspect, the instant invention relates to an isolated nucleic acid sequence able to reduce and/or suppress and/or prevent the presence, or the level expression and/or activity of a peptide according to the invention, as a medicament for restoring sensitivity of an hypoxic apoptosis-resistant cancer cell to an apoptosis-inducing agent for the treatment of cancer.

Within the invention, the term "prevent" intends to refer to the reducing of a risk of occurrence of an event.

According to one of its advantage, the instant invention may be useful in the diagnosis of low-responsive patient or patient resistant to an anticancer treatment and to provide to said diagnosed patient a more efficient treatment.

According to another of its advantage, the instant invention allows the identification of novel active anticancer agents for the treatment of cancer and the prevention, the reduction and/or the suppression of the apoptosis resistant phenotype of cancer cells.

According to another of its advantage, the instant invention allows to tailor the cancer treatment of a patient by following the risk of occurrence of apoptosis-resistant cancer cells and to provide an opportunity to switch in time to a cancer treatment allowing reducing, suppressing or preventing such risk.

### {BRIEF DESCRIPTION OF THE DRAWING}

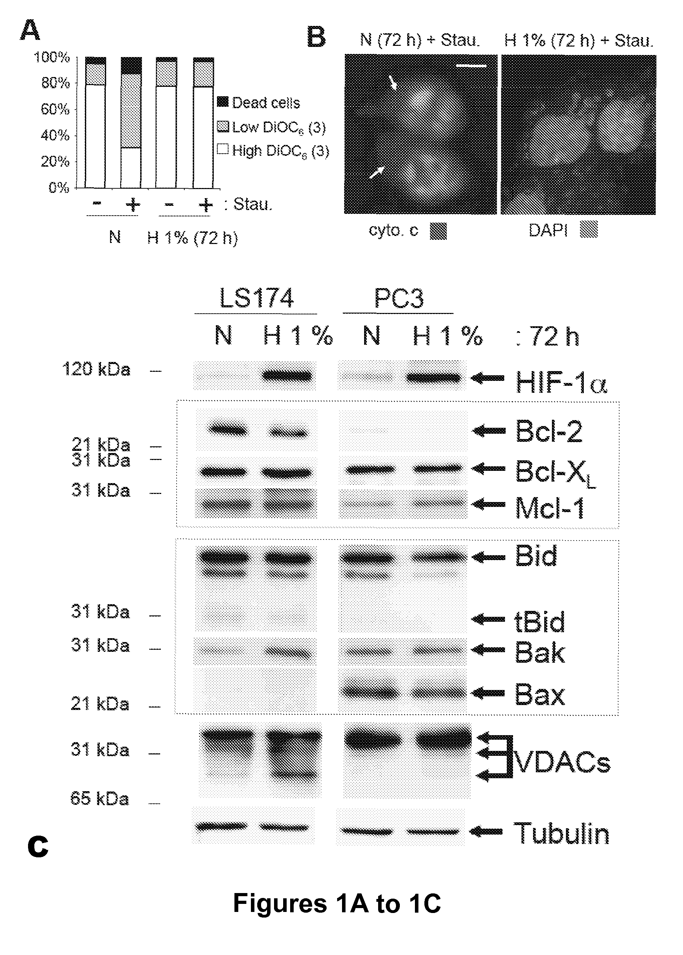

Figure 1. Involvement of anti- and pro-apoptotic proteins in the blockade of cytochrome c release
   (A) Flow cytometric analysis of ΔΨ of LS174 cells in normoxia (N) or hypoxia (H 1%) without (-) or with (+) staurosporine (Stau); mean of 3 experiments. Staurosporine was added for 18 h at 1 µM.
   (B) Immunofluorescence of cyto. c of LS174 cells in normoxia (N) or hypoxia (H 1%) with staurosporine, bar 7.3 µm.
   (C) Immunoblot of the expression of the indicated anti- and pro-apoptotic proteins and VDAC in LS174 and PC3 cells in normoxia (N) or hypoxia (H 1%, 24 h or 72 h). Tubulin is shown as a loading control.

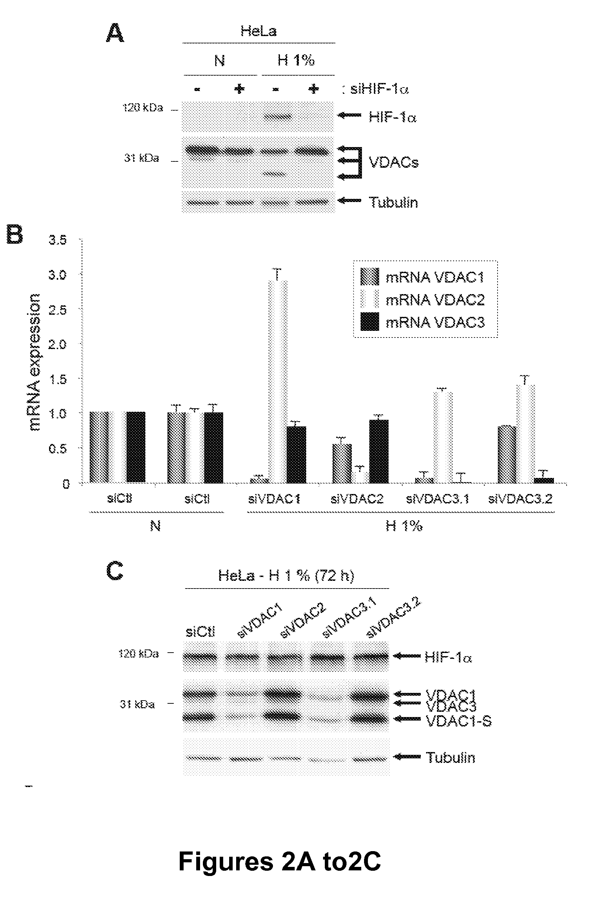

**Figure 2****.** Hypoxia induces a HIF-1-dependent short form of VDAC1 (VDAC1-S)
   (A) Induction in hypoxia of the short form of VDAC is dependent on HIF-1α. Immunoblot of the expression of HIF-1α and VDAC in HIF-1α silenced HeLa cells in normoxia (N) or hypoxia (H 1%) in the absence (-) or presence (+) of HIF-1α siRNA. Tubulin is shown as a loading control.
   (B) Level of expression of the mRNA of VDAC1-3 in normoxia and hypoxia in HeLa cells. Level of expression of VDAC1-3 after transfection with control (siCtl) or VDAC1 (siVDAC1), VDAC2 (siVDAC2) or VDAC3 (siVDAC3.1 or siVDAC3.2) siRNA.
   (C) Immunoblot to HIF-1α and VDAC (ab15895) in control (siCtl) or VDAC1 (siVDAC1), VDAC2 (siVDAC2) or VDAC3 (siVDAC3.1 or siVDAC3.2) silenced HeLa cells in hypoxia (H 1%, 72 h). The signal is normalized to that oftubulin.

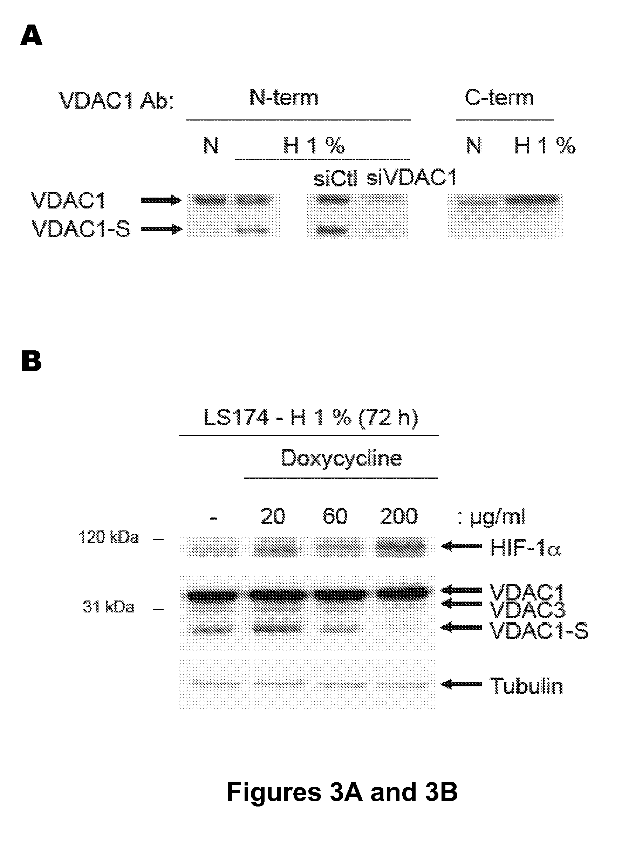

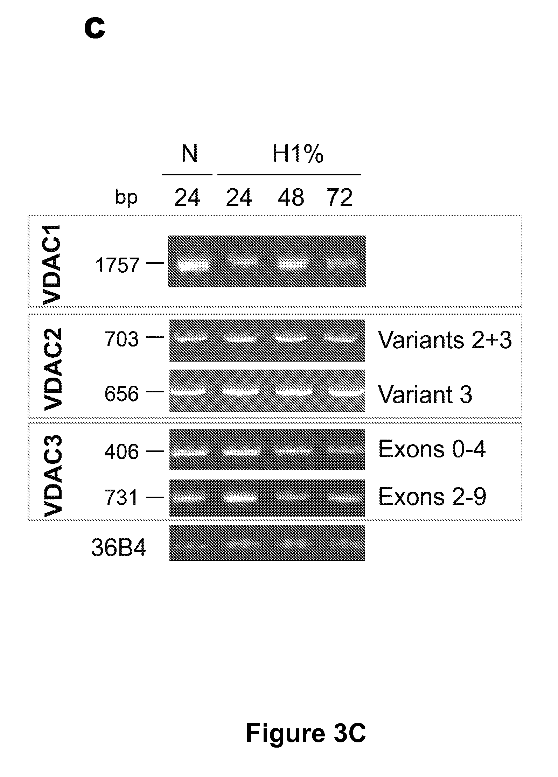

**Figure 3****.** Hypoxia induces short form of VDAC1 (VDAC1-S)
   (A) Immunoblot to VDAC1 using antibodies to the N- or C-terminus of VDAC1 of extracts of HeLa cells incubated in normoxia (N) or hypoxia (H) transfected or not with a control siRNA or a siRNA to VDAC1.
   (B) Immunoblot to HIF-1α and to VDAC (total antibody) of LS174 cells incubated in hypoxia for 72 h. Doxyclycine was added at the indicated concentrations for the first 24 h of hypoxia. Tubulin is shown as a loading control.
   (C) Semi-qPCR of the three isoforms of VDAC.

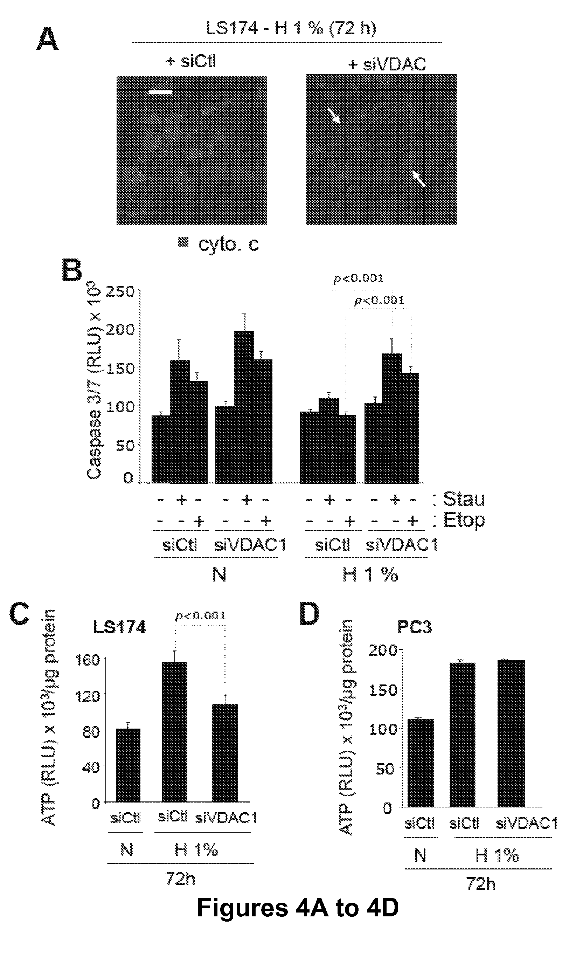

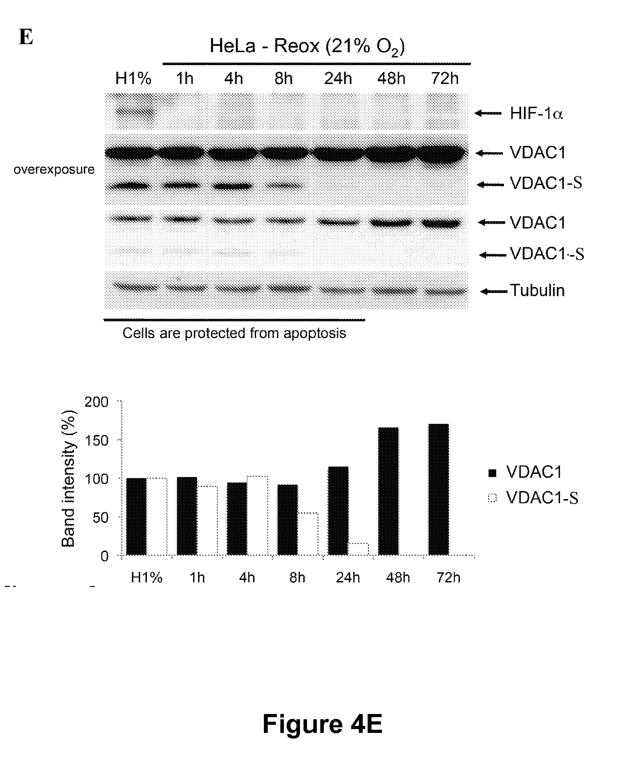

**Figure 4****.** Hypoxic induction of VDAC1-S protects cells with enlarged mitochondria from apoptotic stimuli
   (A) Immunofluorescence of cyto. c in LS174 cells silenced for VDAC1 (siVDAC1) in hypoxia (H 1%, 72 h). Arrows indicate fragmented mitochondria; bar 7.3 µm.
   (B) Caspase activity of control and VDAC1 silenced LS174 cells (n=8, 2 experiments, * *p* < 0.001) in normoxia (N) or hypoxia (H 1%) for 72 h without (-) or with (+) staurosporine (Stau) or etoposide (Etop.) for the last 4 h.
   (C) ATP production of LS174 cells silenced (siVDAC1) or not (siCtl) for VDAC1 (n=8, 2 experiments **p* < 0.001) in normoxia (N) or hypoxia (H 1%).
   (D) ATP production of PC3 cells silenced (siVDAC1) or not (siCtl) for VDAC1 (n=8, 2 experiments **p* < 0.001) in normoxia (N) or hypoxia (H 1%).
   (E) Immunoblot to HIF-1α or VDAC1 of HeLa cells incubated first in hypoxia and then reoxygenated for the indicated times. (top panel). Cells retained protection from apoptosis up to 24h of reoxygenation. B-tubulin is shown as a loading control. (bottom panel) Histograms show quantification of the intensity of the signal to VDAC1-L and VDAC1-S.

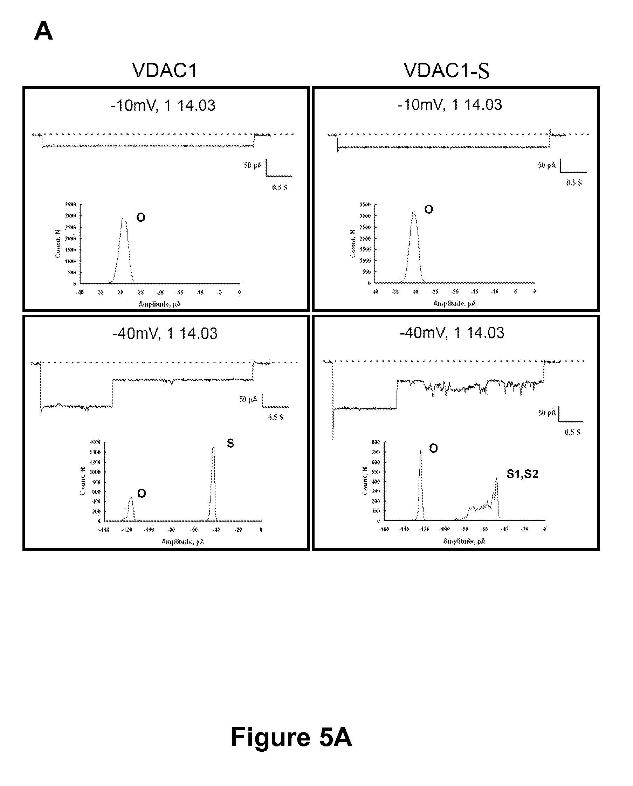

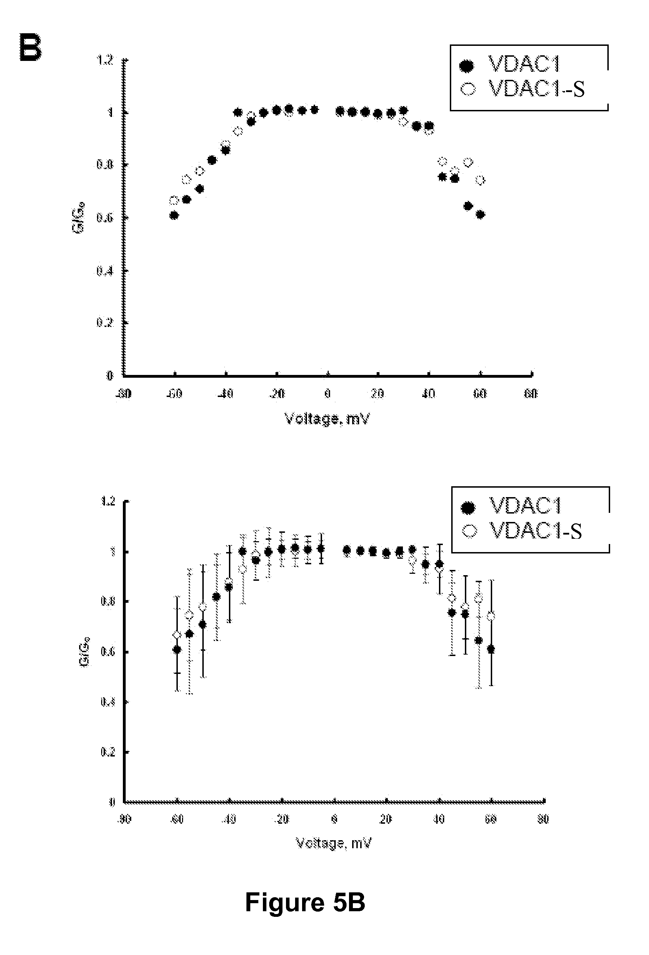

**Figure 5****.** VDAC1-S channel activity and binding of Bcl-xL is identical to that of VDAC1
   (A) Channel activity of bilayer-reconstituted purified VDAC1-L and VDAC1-S. The VDAC1-S protein function as a channels and shows voltage-dependence conductance as does intact VDAC1. Current through bilayer-reconstituted VDAC1 or VDAC1-S in response to a voltage step from 0 to -10 (top panels) or to -40mV (bottom panels) was recorded. The dashed lines indicate the zero current level. In each panel, the total current amplitude histogram traces (in the same recording), showing the relative occupancy of the open state (*O*) and closed sub-state (*S*) or, for VDAC-AC, of 2 or more substates (*S1* and *S2*) during a 4s recording.
   (B) Voltage-dependence of VDAC1-S is the same as VDAC1-L. Currents through the VDAC1 channel in response to a voltage step from 0mV to voltages between -60 to +60mV were recorded. Relative conductance was determined as the ratio of conductance at a given voltage (G) to the maximal conductance (Go). A representative result of five similar experiments is shown in top panel and an average of 5 experiments in bottom panel in which each voltage represents the average of 3-6 swipes.

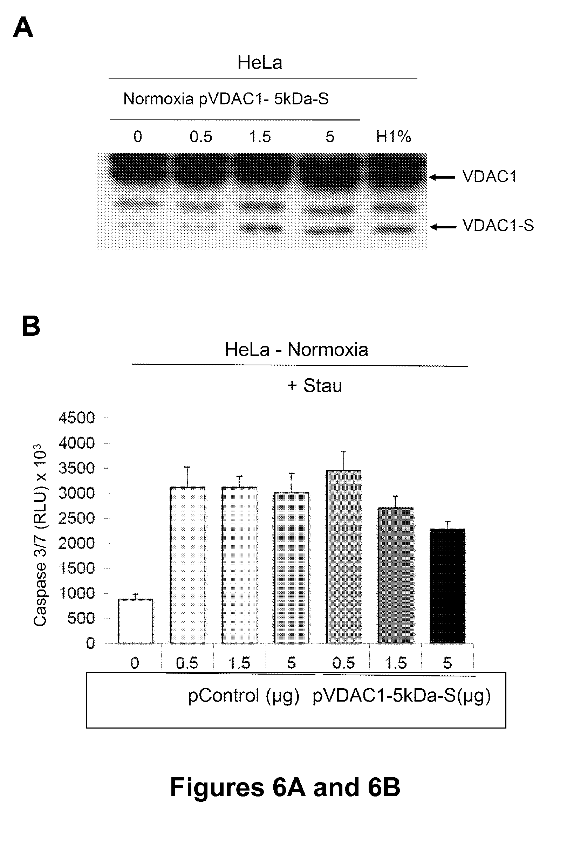

**Figure 6****.** Transfection and expression of VDAC1-S into normoxic HeLa cells.
   (A) A plasmid pVDAC1-5kDa S encoding a truncated form of VDAC1-L, VDAC1-S as set forth by SEQ ID NO: 4, was constructed and transfected into HeLa cells at different concentrations (0; 0.5; 1.5 and 5 µg of plasmid). The plasmid at 1.5 and 5 µg of plasmid allows an expression of VDAC1-S tantamounts to the level of expression obtained with cells placed in hypoxic conditions.
   (B) Caspase activity of control and VDAC1-S transfected HeLa cells (0.5; 1.5; and 5 µg of plasmid) (n= 4 experiments, * *p* < 0.001) in normoxia (N) for 72 h without (-) or with (+) staurosporine (Stau) for the last 4 h.

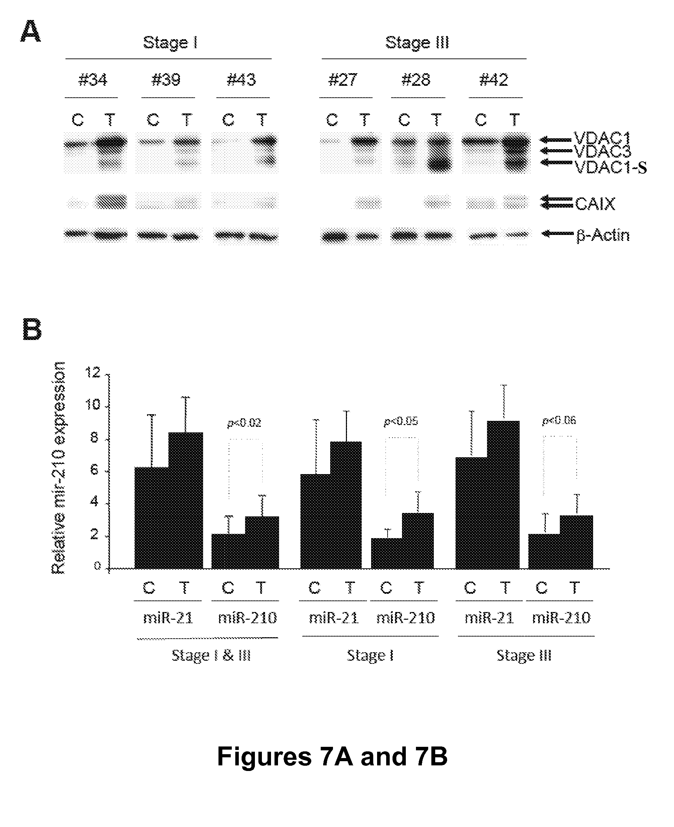

**Figure 7****.** The ratio of VDAC1-S to VDAC1 is higher in tumor tissue compared to control tissue and proportionately more stage III tumors had a high ratio.
   (A) Representive immunoblots to VDAC of extracts of healthy control (C) and tumor (T) tissue from six individual lung cancer patients with either early-stage I or late-stage III tumors. CAIX was used as an indicator of the hypoxic signature of tumors. β-actin is shown as a loading control.
   (B) The fold induction of mir-21 and mir-210 was determined for control and tumor tissues from patients with stage I and III tumors. The level of mir-21 is an indicator of the quality of the miRNA in extracts while mir-210 is a known hypoxia-induced miRNA.

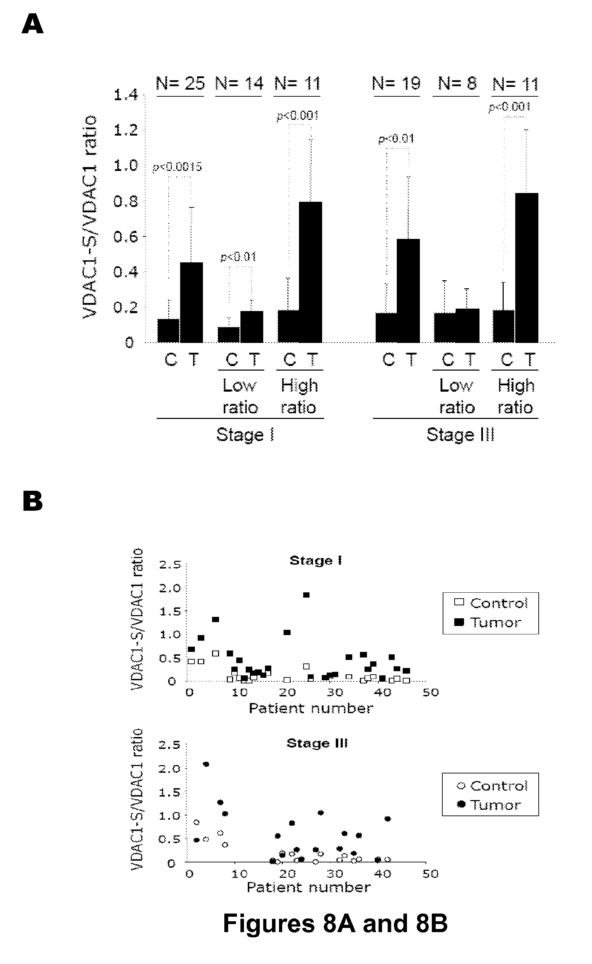

**Figure 8****.** The ratio of VDAC1-S to VDAC1 is higher in tumor tissue compared to control tissue and proportionately more stage III tumors had a high ratio.
   (A) Ratio of the intensity of the immunoblot signal of VDAC1-S to VDAC1 determined with GeneTools software from Syngene for healthy (C) and tumor (T) tissue from lung cancer patients with either stage I or III tumors.
   (B) Ratio of the intensity of the immunoblot signal of VDAC1-S to VDAC1 for each individual patient, determined with the GeneTools software from Syngene for healthy
   (C) and tumor (T) tissue. Lung cancer patients with stage I tumors (upper graph) and stage III tumors (lower graph).

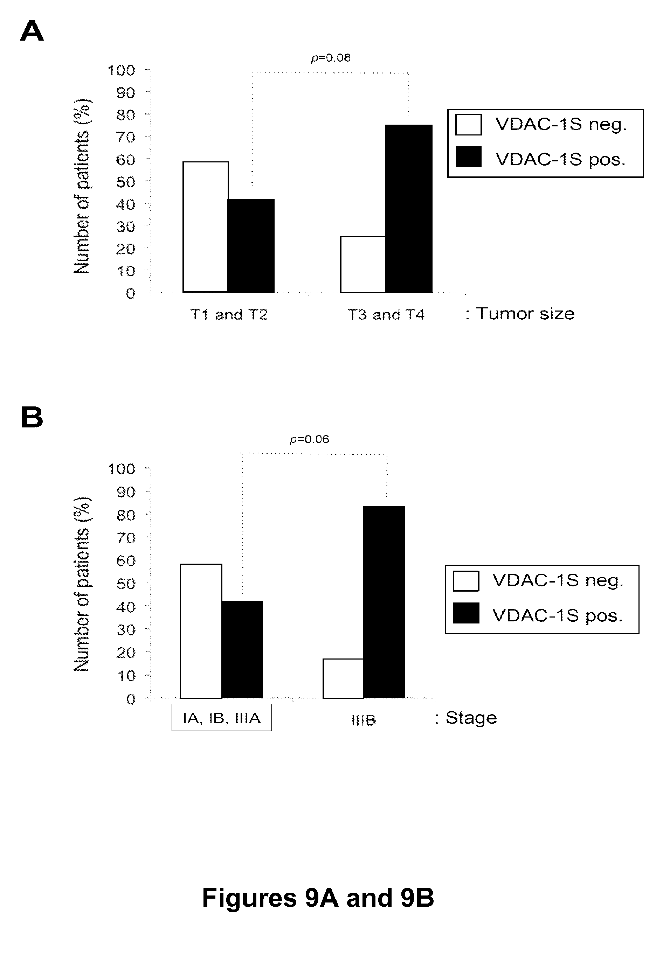

**Figure 9****.** The ratio of VDAC1-S to VDAC1 is higher in tumor tissue compared to control tissue and proportionately more stage III tumors had a high ratio.
   (A) Evaluation of the number of lung cancer patients either negative or positive for VDAC1-S, respectively (VDAC1-S neg) and (VDAC1-S pos), with small- (T1 and T2) or large- (T3 and T4) sized tumors.
   (B) Evaluation of the number of lung cancer patients either negative or positive for VDAC1-S, respectively (VDAC1-S neg) and (VDAC1-S pos), with stages IA - IB - IIIA or stage IIIB tumors.

**Table 1:** Patient and tumor characteristics.

**Table 2:** Comparison of the characteristics of the patients and their tumors with VDAC1-S detection.

### {DESCRIPTION OF EMBODIMENT}

The present invention relates to the identification and the use of a short, C-terminally truncated, isoform of voltage dependent anion channel 1 (VDAC1-S) as a novel cell marker useful for characterizing an apoptosis-resistance phenotype of hypoxic cancer cells.

VDAC1 belongs to a group of mitochondrial membrane channels involved in translocation of adenine nucleotides through the outer membrane. VDACs are small, functionally conserved proteins found in the outer mitochondrial membrane of all eukaryotes. These channels may also function as a mitochondrial binding site for hexokinase and glycerol kinase. VDAC1 appears to play a role in the maintenance of the mitochondrial transmembrane potential. The structure of VDAC1 has been resolved and shows a transmembrane channel of 19 amphipathic beta strands and a mobile N-terminal alpha helix located inside the pore that regulates the opening/closing (Hiller, 2010, Science 321, 1206-1210). The N-terminal domain has been reported to regulate apoptosis, by binding anti-apoptotic proteins, and to control the release of cyto. c (Abu-Hamad, 2009, J Cell Sci, 122, 1906).

Mammalian VDACs consist of three family members encoded by three separate genes: *vdac1*, *vdac2* and *vdac3* (Lemasters, 2006, Biochim Biophys Acta, 1762, 181-190; Rostovtseva, 2005, J Bioenerg Biomembr, 37, 129-14; Shoshan-Barmatz, 2006, Curr Pharm Des, 12, 2249-2270). The three isoforms of VDACs have substantial homology. Eight transcripts have been described for VDAC1 (ENST00000395047). Three code for the same protein of 283 amino acids, one for a protein missing the 99 C-terminal amino acids, one for a protein missing 127 amino acids in the center and the other three are non protein coding. An alternative variant of the VDAC3 isoform containing an insertion of an ATG was identified from the cDNA (Sampson, J Biol Chem, 273, 30482-30486).

VDAC1 has been characterised, up to the present invention, as a single isoform, designed in the present specification VDAC1-L, and may be represented by the polypeptide as set forth in SEQ ID NO: 3, or analog, or variant, thereof having at least an identity of sequence of at least 90% and a similar biology activity.

The present invention has identified a novel peptide, namely a C-terminally truncated form of VDAC1-L, VDAC1-S.

The molecular weight of a peptide of the invention, or VDAC1-S, estimated from gel polyacrylamide electrophoresis migration, or SDS-PAGE immunoblots is around 5 kDa smaller than VDAC1-L.

A peptide of the invention, or VDAC1-S, may be recognized by an antibody able to specifically bind to an amino acids sequence corresponding to amino acids residues 150-250 of the amino acids sequence as set forth in SEQ ID NO: 3, but not by an antibody able to bind to an amino acids sequence corresponding to amino acids residues 234 to 283 of the amino acids sequence SEQ ID NO: 3.

Also, a peptide of the invention, or VDAC1-S, may be recognized by an antibody able to specifically bind to an amino acids sequence corresponding to amino acids residues 1-30 of the amino acids sequence set forth by SEQ NO:3.

As suitable example of third antibody useful for the invention, one may mention the antibody AP6627a from ABGENT.

Preferably, a peptide of the invention may be resolved from a cellular extract by SDS-PAGE and identified through imunoblotting on a PVDF membrane, for example blocked with non-fat milk, preferably 5%, in a buffer, preferably a TN buffer (50 mM Tris-HCl Ph 7.4, 150 Mm NaCl), in the presence of the necessary antibodies, primary and secondary antibodies. A peptide of the invention, or VDAC1-S, is a C-terminaly truncated form of VDAC1-L for example as set forth by SEQ ID NO: 3, or analog thereof having at least an identity of sequence of at least 90% and a similar biology activity.

A peptide of the invention, or VDAC1-S, may comprise less than about 33 to about 53, preferably 35 to about 51, preferably 37 to about 49, preferably 39 to about 47, preferably 41 to about 45 and more preferably less than 44 contiguous C-terminal amino acids from the amino acid sequence of VDAC1-L.

A peptide of the invention, or VDAC1-S, may be represented by an amino acids sequence consisting in 230 to 250 contiguous N-terminal amino acids of SEQ ID NO: 3, and analog thereof having at least an identity of sequence of at least 90% and a similar biology activity.

Preferably, a peptide of the invention may be represented by an amino acid sequence consisting in 232 to 248, preferably 234 to 246, preferably 236 to 244, preferably 238 to 239, and more preferably 239 contiguous N-terminal amino acids of SEQ ID NO: 3, and analog thereof having at least an identity of sequence of at least 90% and similar biology activity.

More particularly, a peptide of the invention may be represented by an amino acids sequence as set forth in SEQ ID NO: 4, or analog thereof having at least an identity of sequence of at least 90% and a similar biology activity.

Preferably, an analog of a peptide of the invention may have an identity of sequence of at least 95%, and more preferably of at least 99% and a similar biology activity.

A peptide of the invention, or VDAC1-S, is generated without detectable corresponding coding mRNA.

The method to be performed in connection with a detection of mRNA may be a RT-PCR. Such RT-PCR may performed with the primers 5'-gccgctcgctcggctccgct-3' (SEQ ID NO: 7) and 5'-gcattgaaggggaggatct-3' (SEQ IDN NO: 8) and 30 cycles at 94°C for 30 seconds, then 55°C for 1 minute, and then 72°C for 1 minute. In such conditions, not mRNA coding for a peptide of the invention is detected. The PCR products may then be subjected to an electrophoresis gel migration operated on an 1.5 or 3% agarose gels containing a detectable marker for nucleic acid, such as a fluorescent marker, for example ethidium bromide.

The generation of a peptide of the invention may be prevented by inhibitor of matrix metalloprotease, as for example doxycyclin. A peptide of the invention may be generated by an enzymatic cleavage resulting in the removing of a C-terminal portion of VDAC1 comprising from 33 to 53, in particular from 35 to 51, in particular from 37 to 49, in particular from 39 to 47, in particular from 41 to 45, in particular from 43 to 44, and preferably from about 44 amino acids starting from the last G-terminal amino acid.

The long isoform of VDAC1 appears to be constitutively expressed in cells whereas VDAC1-S appears to be expressed, as observed by the inventors, upon induction after at least 48 hours, and more preferably after 72 hours of hypoxic condition.

Given that the long isoform VDAC1 is constitutively expressed and that VDAC1-S is expressed upon induction, one may understand that the determination of VDAC1-S need not to be specifically operated on VDAC1-S itself, but may be operated on VDAC1 constituted of VDAC1-L and VDAC1-S. An observed difference between the determinations from two samples will be then informative on VDAC1-S.

According to a preferred embodiment, the cell marker VDAC1-S is human VDCA1-S.

According to another embodiment, the invention also relates to a polypeptide, in particular an isolated polypeptide, represented by an amino acids sequence consisting in 33 to 53, in particular 35 to 51, in particular 37 to 49, in particular 39 to 47, in particular 41 to 45, in particular 43 to 44, and preferably 44 contiguous C-terminal amino acids of VDAC1-L, and in particular may be of the amino acids sequence as set forth in SEQ ID NO: 5, or an analog thereof having at least an identity of sequence of at least 90%, and a similar biology activity.

According to another embodiment, the invention relates to an isolated nucleic acid sequence coding for a polypeptide of the invention, or an analog thereof having an identity of sequence of at least 90%, and a similar biology activity.

An isolated nucleic acid sequence of the invention may be obtained, for example, from the nucleic acid sequence coding for VDAC1-L, SEQ ID NO: 1, modified to remove the nucleic acids coding for the 33 to 53 C-terminal amino acids, and preferably coding for the 33 to 53, in particular for the 35 to 51, in particular coding for the 37 to 49, in particular coding for the 39 to 47, in particular coding for the 41 to 45, in particular coding for the 43 to 44, and preferably coding for the 44 C-terminal amino acids.

Those nucleic acids sequences may be removed by using any suitable known restriction enzyme.

Alternatively, an isolated nucleic acid sequence of the invention may be obtained from a cDNA coding for VDAC1-L and subjected to a PCR with a suitable primers designed to amplify specifically the sequence of interest, in particular SEQ ID NO: 2. The cDNA coding for VDAC1-L may be obtained from any cells expressing this protein by reverse transcription of the mRNA coding for this protein. Those biological molecular techniques are known from the skilled artisan, and are described in the handbook "Molecular cloning: A Laboratory Manual" (Sambrook J *et al.*, 2000, 3^{rd} Ed., Cold Spring Harbour Laboratory Press).

As example of a suitable primers for amplifying an isolated nucleic sequence of the invention from a cDNA encoding for VDAC1-L, in particular as set forth in the nucleic acid sequence SEQ ID NO:1, one may mention the forward and reverse primers as set forth in SEQ ID NO: 21 and SEQ ID NO: 22.

An isolated nucleic acid sequence of the invention may be represented by a nucleic acids sequence as set forth by SEQ ID NO: 2, or an analog thereof having an identity of sequence of at least 90%, and a similar biology activity.

A nucleic acid sequence coding for a polypeptide of the invention may be advantageously used to establish transfected cells or non human transgenic animals able to express VDAC1-S, which may be useful for establishing screening tools for novel anticancer active agent.

Accordingly, an isolated nucleic acid sequence of the invention may be subcloned into any vectors suitable for the expression of the protein of interest into a suitable host cell. As example of a suitable vector of expression, one may mention the plasmid pcDNA 3.3-TOPO TA.

The obtaining of such transfected cells or non-human transgenic animals may be performed by using any known methods in the art comprising the use of any suitable expression vector containing a nucleic acid sequence of the invention.

Also, the invention relates an isolated vector comprising a nucleic acid of the invention as well as to an isolated host cell comprising a nucleic acid of the invention.

An identity of sequence of two polynucleotides or two polypeptides may be visually determined or may be determined by any methods usually implemented in the art, such as a BLAST algorithm implemented software available on Pubmed website (www.ncbi.nlm.nih.gov).

According to the invention, a biological activity similar to VDAC1-S with respect to an analogue thereof intends to refer to an activity of maintenance of the mitochondrial transmembrane potential in hypoxia condition and/or the switching of the tubular mitochondria phenotype toward enlarged mitochondria phenotype and/or the resistance of hypoxic cancer cells to apoptosis-inducing agent.

Also, a similar biological activity with respect to VDAC1-S or VDAC1-L may be their channel activity which may be assessed as detailed in the examples.

In particular, VDAC1-S or VDAC1-L may be transferred into a planar lipid belayer and its channel activity may be determined by voltage-clamping.

The determination of VDAC1-S may be carried out in an isolated biological sample, and in particular in an isolated cell sample.

As used herein, the term "biological sample" includes a sample from any body fluid or tissue (e.g. serum, plasma, blood, cerebrospinal fluid, urine, kidney tissue) liable to contain VDAC1-S or cancer cells.

An isolated biological sample or an isolated cell sample may be a biopsy taken from a tissue, preferably taken from a tumour or a cancerous tissue.

In one embodiment, an isolated biological sample or an isolated cell sample may be obtained from an individual suspected of having a cancer resisting to a cancer treatment.

VDAC1-S is referred to as differential expressed in one sample as compared to another sample when a method used for determining VDAC1-S provides a difference on the presence or absence, or on a level of expression or activity of VDAC1-S when applied to the two samples.

VDAC1-S is referred to as increased in a first sample if the method for determining VDAC1-S indicates that the level of expression or activity of VDAC1-S is higher in the first sample than in a second sample, or if VDAC1-S is detectable in the first sample but not in the second sample.

Conversely, VDAC1-S is referred to as decreased in the first sample if the method for determining VDAC1-S indicates that the level of expression or activity of VDAC1-S is lower in the first sample than in the second sample, or if VDAC1-S is detectable in the second sample but not in the first sample.

When a ratio VDAC1-S/VDAC1 is used, the determination of a level of expression or activity of VDAC-1 is carried out on the same sample on which the determination with respect to VDAC1-S has been performed. The ratio VDAC-1S/VDAC1 may be advantageously used to normalized the determination carried out with respect to VDAC1-S.

In some embodiments, the presence or absence, or level of expression and/or activity of VDAC1-S may be compared to the presence or absence, or level of expression and/or activity of VDAC1-S in a different tissue or biological "compartment".

The determination of the presence or absence, level of expression and/or activity of VDAC1-S as well as VDAC-1, or VDAC1-L, may be carried out by any conventional technique of cell biology, analytical biochemistry, or molecular biology techniques within the art useful for evaluating or quantifying the presence or absence, or level of expression or activity of a cell constituent such as a polypeptide.

The determination may be operated directly on a sample or after extracting VDAC1-S from a sample.

According to one embodiment, a determination may be carried out by a method chosen from mass spectrometry such as LC-MS, GC-MS, chromatographic separations such as one or 2-D gel separations, binding assays such as immunoassays or hybridization, enzyme assays such as ELISA, or competitive inhibition assays.

Preferably, a determination may be carried out by a method chosen from Western-blot, immunoassays ELISA, fluorescence microscopy, flow cytometry, gel electrophoresis, or column chromatography.

According to an embodiment, the determination of presence or absence or level of expression and/or activity of a peptide relating to VDAC1-S may be carried out by any method known in the art. For example, the determination of a peptide relating to VDAC1-S may be based on the functional or antigenic characteristics of the polypeptide. The determination may be operated directly on the sample or after extraction.

Methods for polypeptide determination include immunoassays, Western-blot, ELISA, fluorescence microscopy or flow cytometry.

According to one embodiment, determination may utilize staining of cells or histological sections, performed in accordance with conventional methods.

Alternatively, polypeptide determination may include polypeptide extraction from a cell or tissue sample, followed by binding of a labelled probe (e.g., an antibody) specific for the target polypeptide, and detection of the probe. The label group can be a radioisotope, a fluorescent compound, an enzyme, or an enzyme co-factor.

According to one embodiment, polypeptide determination may be assessed by gel electrophoresis or column chromatography.

In a preferred embodiment, a peptide relating to VDAC1 may be determined by one- or two-dimensional gels, optionally comprising a step of transfer on a blot, such as Western blot. Such technique allows the proteins to be separated and further identified and quantified for example by silver staining or antibodies tagged with fluorescence or radioactive label.

Western blot may be advantageously performed on protein gels or protein spots on filters, using detection system specific for peptide relating to VDAC1-S, and may conveniently use a labelling method as described for the sandwich assay.

According to another embodiment, a peptide relating to VDAC1-S may be determined using antibodies or other specific biding members added to a biological sample or to a cell sample or a lysate thereof and incubated for a period of time sufficient to allow binding to VDAC1-S, for example at least about 10 minutes. Antibodies may be labelled with radioisotope, enzymes, fluorescent molecules, chemiluminescent molecules, or other labels for direct detection. Alternatively, a second stage antibody or reagent may be used to amplify the signal. Such reagents are well-known in the art. For example, a primary antibody may be conjugated to biotin, with horse radish peroxydase-conjugated avidin added as a second stage reagent. Final detection may use a substrate that undergoes a colour change in the presence of the peroxydase. A second stage antibody may also, for example, be labeled with a fluorescent or a radioisotopic molecule. The absence or presence or relative amount of antibody binding may be then determined by various methods, including flow cytometry, fluorescence microscopy, radiography, scintillation counting, etc.

Also a conventional sandwich type assay may be used. For example, a sandwich assay may first attach VDAC1-S specific antibodies to an insoluble surface or support. In such method, a series of standards containing known a concentration of VDAC1-S may be assayed in parallel with the samples to serve as controls. The incubation time should be sufficient for binding, generally from 0.1 to 3 hours is sufficient. After incubation, the insoluble support is generally washed and a solution containing a second antibody is applied, which may be labelled to facilitate direct or indirect detection and/or quantification of binding. Examples of labels include radiolabels such as ³H or ¹²⁵I, fluorescent molecules, dyes, beads, chemiluminescent molecules, colloidal particles, and the like. Examples of labels permitting indirect measurements include enzymes where the substrate may be provided for a coloured or fluorescent product.

In one embodiment, peptides relating to VDAC1-S may be determined using a standard immunoassay, such as sandwiched ELISA using matched antibody pairs and fluorescent or chemiluminescent detection. Commercially available or custom monoclonal or polyclonal antibodies may be used.

In some cases, a competitive assay may be used. In such assay, a competitor to VDAC1-S is added to the isolated biological sample or the isolated cell or lysate thereof. The competitor and VDAC1-S compete for binding to a specific binding partner. Usually, the competitor molecule may be labelled and detected as previously described, whereas amount of competitor binding will be proportional to the amount of VDAC1-S presence. The competitor molecule will be from about 10 times the maximum anticipated VDAC1-S concentration to about equal concentration in order to make the most sensitive and linear range of detection.

In some embodiments, the methods may be adapted for use *in vivo*, e.g. to locate or identify sites where apoptosis-resistant hypoxic cancer cells are present.

In these embodiments, a detectably labelled moiety, e.g. an antibody, which is a specific for VDAC1-S is administered to an individual (e.g. by injection), and labelled cells are located using standard imaging techniques, including but not limited to magnetic resonance imaging, computed tomography scanning, and the like. In this matter, apoptosis-resistant hypoxic cancer cells are differentially labelled.

The present invention relates to methods for determining an apoptosis-resistance phenotype of at least one isolated hypoxic cancer cell suspected to be apoptosis-resistant, or for assessing responsiveness of an individual suffering from a cancer to a cancer treatment, or for staging a cancer in an individual having a cancer as previously indicated.

The determination of VDAC1-S may be performed as previously described.

In a method for determining an apoptosis-resistance phenotype of at least one isolated hypoxic cancer cell suspected to be apoptosis-resistant, an increase of said level of expression and/or activity relative to the control value or range detects an apoptosis-resistance phenotype.

In a method for assessing responsiveness of an individual suffering from a cancer to a cancer treatment, a presence or an increase of a level of expression and/or activity of a peptide of the invention in the second biological sample relative to the first biological sample may be indicative of a low-responsiveness of the individual to said cancer treatment.

The cancer may therefore be categorized as resistant cancer.

Alternatively, an absence or a decrease of a level of expression and/or activity of a peptide of the invention in the second biological sample relative to the first biological sample may be indicative of a responsiveness of the individual to said cancer treatment, or that the cancer condition is or become responsive to the cancer treatment.

As will be apparent to those of ordinary skill in the art, the invention is not limited to making an initial diagnosis of resistant cancer or the responsiveness of a cancer to a given cancer treatment, but also is applicable to confirming a provisional diagnosis of resistant cancer or "ruling out" such a diagnosis.

Furthermore, a presence or an increased level of expression and/or activity of VDAC1-S in a sample obtained from an individual suspected of having a cancer resistant to a cancer treatment, or at risk for developing a cancer resistant to a cancer treatment, is indicative that the individual has or is at risk for developing a resistant cancer.

According to another embodiment, the invention also provides a method for determining individual's risk of developing a cancer resistant to a cancer treatment, the method comprising obtaining a biological sample from an individual, determining the presence or absence, or level of expression and/or activity of VDAC1-S in the sample, and comparing the result to the presence or absence, or level of expression and/or activity of VDAC1-S in a sample obtained from an individual having not a cancer resistant to a cancer treatment, or to a control value or range, wherein a presence or an increase of level of expression or activity of VDAC1-S is correlated with the risk of a resistant cancer.

According to another embodiment, the invention also provides a method for determining a stage or severity of a cancer resistant to a cancer treatment.

Such a method may comprise obtaining a biological sample from an individual having a cancer, determining a presence or absence, or level of expression and/or activity of VDAC1-S in the sample, and comparing the result to the presence or absence or level of expression or activity of VDAC1-S in a sample obtained from an individual having not a resistant cancer, or to a control value or range, wherein an increase or decrease of VDAC1-S is correlated with a stage or severity of the disease.

A resistant cancer may be diagnosed in an individual if a level of expression or activity of VDAC1-S in the individual sample is statistically more similar to the level of expression or activity of VDAC1-S that has been associated with a resistant cancer than the level of expression or activity of VDAC1-S that has been associated with the control.

A method of the present invention may be used to make a diagnosis of a resistant cancer, independently from other information such as the individual symptoms or the results of other clinical or paraclinical tests. However, a method of the present invention may be used in conjunction with such other data points.

In another aspect, the invention provides a method for monitoring the progression of a cancer resistance to a cancer treatment in an individual who has a cancer.

Such method may comprise obtaining a first biological sample from an individual having a cancer and subjected to a cancer treatment at time to, determining a presence or absence, or level of expression and/or activity of VDAC1-S in the sample, and comparing the result to a presence or absence, or level of expression and/or activity of VDAC1-S in a second biological sample obtained from said individual at a later time t₁, wherein a presence or absence or an increase or decrease of VDAC1-S is correlated with progression of the cancer resistance to a cancer treatment.

Depending upon the difference between the determinations, it can be seen whether the VDAC1-S is present or absent or whether its level of expression or activity has increased, decreased, or remained constant over time.

A further deviation of VDAC1-S in a direction indicating resistant cancer, or the measurement of additional increased VDAC1-S, would suggest a progression of the cancer resistance to the cancer treatment during the interval.

Subsequent sample acquisitions and determination may be performed as many times as desired over a range of times t₂ to tₙ.

The ability to monitor a subject by making serial VDAC1-S level of expression and/or activity determinations over time may be used to follow a progression of the cancer resistance, to predict exacerbations or to indicate the clinical course of the cancer resistance.

In a method for staging a cancer in an individual having a cancer, a presence or an increase of said level of expression and/or activity relative to the control value or range is indicative of advanced stage cancer.

A cancer to be staged may be chosen from breast cancer, head-and-neck cancer, liver cancer, ovarian cancer, pancreatic cancer, prostate cancer, renal cancer, skin cancer, testis cancer, lung cancer, cervical cancer or brain cancer, andin particular may be a lung cancer.

More particularly, VDAC1-S may be a biomarker of advanced cancer or large tumor with respect to lung cancer. In particular, VDAC1-S may a biomarker of stage IIIB, and further, of lung cancers.

VDAC1-S may be also used as a biomarker of cancer or tumor progression.

According to another embodiment, a method of the invention may further comprise a step of determining an absence or a presence of mitochondria of enlarged phenotype.

As observed by the inventors, expression of VDAC1-S allows the mitochondrial transmembrane potential of cancer cells to be maintained in hypoxic conditions and to modify the morphology of tubular mitochondria toward enlarged mitochondria phenotype.

According to one embodiment, a use of VDAC1-S according to the invention may be associated with the determination of the presence or the absence of mitochondria of enlarged phenotype.

In particular, a presence, or an increased level of expression and/or activity of VDAC1-S may be associated with a presence of mitochondria of enlarged phenotype.

Mitochondria of enlarged phenotype present an increased size with respect to mitochondria of tubular phenotype which are usually observed in normal and healthy cells.

The determination of the presence or absence of mitochondria of enlarged phenotype may be carried out by any methods of cell biology or cell imaging. For example, one may use direct observation with electron microscopy or fluorescent microscopy after immunolabeling mitochondria with antibodies, for example directed to cytochrome c, tagged with a fluorescent or a chemiluminescent molecule or an enzyme able to convert colorimetric substrate.

An activity, and in particular a biological activity, relating to VDAC1-S may be determined by determining the presence or the absence of mitochondria with an enlarged phenotype in an isolated biological sample or an isolated cell sample as previously indicated, or the mitochondrial transmembrane potential of isolated hypoxic cells or the resistance to the apoptosis of hypoxic cells.

The mitochondrial transmembrane potential (ΔΨm) may be measured by any conventional techniques known in the art.

For example, the mitochondrial transmembrane potential may be determined by the use of fluorescent molecules which accumulate specifically in the inter membrane space of the mitochondria, such as DiOC₆(3). The fluorescence may be observed by any known fluorescent methods, such as fluorescent microscopy or flow cytometry.

The apoptosis-resistance inducing activity of VDAC1-S may be observed by subjecting isolated cells, and preferably isolated cancer cells, to hypoxic conditions and contacting a first sample of hypoxic cells with an apoptosis-inducing agent, such as etoposide or staurosporine, and comparing the death cells rate or the presence or absence or the level of expression and/or activity of different markers specific to apoptosis in the first sample to a second sample of hypoxic cells not contacted with said agent.

Markers specific to apoptosis, may be, for example, caspase activity, annexin V flip-flop or DNA ladder, and may be detected using any known methods in the art, such as fluorescence microscopy, flow cytometry, ELISA or gel electrophoresis.

In another aspect, the present invention provides a method for screening candidate active agents for use as therapeutic compounds.

According to one embodiment, the present invention provides a method for screening candidate active agents able to restore apoptosis-sensitivity to an apoptosis-resistant hypoxic cancer cell comprising at least the steps of:
a) providing at least one isolated cell able to express VDAC1-S or a non-human transgenic animal able to express VDAC1-S in condition suitable for the expression of VDAC1-S,
b) contacting said isolated cell or said non-human transgenic animal with at least one candidate active agent to be screened,
c) determining in said isolated cell or in said non-human transgenic animal a presence or absence, or level of expression and/or activity of VDAC1-S, and
d) comparing said determined presence or absence, or level of expression and/or activity to a control value or range.

According to another embodiment, the present invention provides a method for screening candidate active anticancer agents comprising at the least the steps of:
a) subjecting at least one candidate active anticancer agent known as having anticancer activity to the screening method as previously defined, and
c) selecting the active candidate anticancer agent able to reduce, suppress and/or prevent presence or an expression and/or an activity of VDAC1-S.

According to another aspect, the instant invention relates to a method for screening active anticancer agents comprising at the least the steps of:
a) subjecting at least one candidate active anticancer agent known as having anticancer activity to a method of the invention for screening candidate active agents, as previously defined, and
b) selecting the candidate active anticancer agent able to reduce, suppress and/or prevent a presence, or an expression and/or an activity of VDAC1-L or VDAC1-S.

Within the invention, the expression "anticancer activity" refers to a property of a physical, chemical or biological agent to stabilize, reduce, suppress and/or prevent the abnormal proliferation and/or invasion of cells *in vivo* or *in vitro*, by inducing apoptosis.

The present invention provides a method for screening candidate active agents able to bind to, to modulate or to mimic the biological activity of polypeptide or polynucleotide relating to VDAC1-S.

Candidate compounds which affect the presence or absence, or the level of expression and/or activity of VDAC1-S may be identified using any suitable methods or techniques known in the art, and in particular in using the methods previously described.

A screening method of the invention may be performed using an *in vitro* model, an isolated cell, a genetically modified cell or animal.

When performed on isolated cells, a method for screening of the invention may be performed on primary, immortalized or tumour cells in which VDAC1-S is either naturally expressed or expressed through introduction through an expression vector using an inducible expression system.

Transgenic animals or cells derived therefrom may also be used in a screening method of the invention. Transgenic animals may be made by any methods known in the art. For example, transgenic animals may be made through homologous recombination where the normal VDAC1 locus is altered. Alternatively, a nucleic acid construct may be randomly integrated into the genome. Vectors for stable generation include plasmids, retrovirus and other animal viruses, YACS, and the like.

In a further additional embodiment, VDAC1-S may be used to screen candidate active agent, for example, in a clinical trial, to determine whether a candidate active agent is effective in preventing and/or treating a resistant cancer.

At time to, a biological sample is obtained from each subject in population of subjects diagnosed with resistant cancer. Next, assays are performed on each subject's sample to measure a presence or absence, or levels of expression and/or activity of VDAC1-S.

Next, a predetermined dose of a candidate active agent is administered to a portion or sub-population of the same subject population. Active agent administration may follow any suitable schedule over any time period. In some cases, varying doses are administered to different subjects within the sub-population, or the candidate active agent is administered by different routes. At time t₁, after active agent administration, a biological sample is acquired from the sub-population and the same assays are performed on the biological samples as were previously performed to obtain determination of presence, absence or values.

As previously indicated, subsequent samples acquisitions and determinations may be performed as many times as desired over a range of times t₂ to tₙ.

In such a study, a different sub-population of the subject population serves as a control group, to which a placebo is administered. The same procedure is then followed for the control group: obtaining the biological sample, processing the sample, and determining VDAC1-S to obtain a determination chart.

Specific doses, delivery routes, time of administration can also be examined. The method is performed by administering the candidate active agent at specified dose, delivery routes or period of time to subjects with resistant cancer; obtaining biological samples from the subjects; determining the presence or absence, or level of expression and/or activity of VDAC1-S in each of the biological samples; and, comparing the determined presence or absence, or level of expression and/or activity of VDAC1-S for each sample with other samples and/or a control value or range.

Typically, the control value or range may be obtained by determining VDAC1-S in the subject before active agent administration. Depending upon the difference between the determined and control value or range, the dose, route of delivery or time of administration may be considered to have an effect on a resistant cancer.

According to one embodiment, the present invention provides an isolated nucleic acid sequence able to reduce and/or suppress and/or prevent the presence or level of expression and/or activity of VDAC1-S as a medicament for restoring sensitivity of a hypoxic apoptosis-resistant cancer cell to an apoptosis-inducing agent for the treatment of cancer.

Also, the invention relates to the use of an isolated nucleic acid sequence able to reduce and/or suppress and/or prevent the presence or level of expression and/or activity of VDAC1-S for the manufacture of a medicament or a pharmaceutical composition for restoring sensitivity of a hypoxic apoptosis-resistant cancer cell to an apoptosis-inducing agent for the treatment of cancer.

A cancer considered by the invention may be chosen from breast cancer, head-and-neck cancer, liver cancer, ovarian cancer, pancreatic cancer, prostate cancer, renal cancer, skin cancer, testis cancer, lung cancer, cervical cancer or brain cancer, andin particular may be a lung cancer.

The nucleic acid may be a gene or an antisense sequence able to control, and in particular to down-regulate, the expression of VDAC1. Such sequences may, for example, be transcribed in the target cell into RNAs complementary to cellular mRNAs and thereby block their translation into protein, according to the technique described in EP 0 140 308. Alternatively, such sequences may be introduced as such in the cells under the form of RNA.

The nucleic acids may be single- or double-stranded, as well as short oligonucleotides or longer sequences.

These nucleic acids may be of human, animal, plant, bacterial or viral origin and the like. They may be obtained by any technique known in the art, and in particular by screening libraries, by chemical synthesis or alternatively by mixed methods including chemical or enzymatic modification of sequences obtained by screening libraries. They may be chemically modified.

A nucleic acid sequence may be chosen from a siRNA, a miRNA, a shRNA, a hybrid sequence or a synthetic or semi-synthetic sequence of oligonucleotide.

More particularly, antisense molecules may be antisense oligonucleotides (ODN), particularly synthetic ODN having chemical modifications from native nucleic acids or nucleic acid constricts that express such antisense molecule as RNA. Antisense molecules may inhibit gene expression through various mechanisms, e.g. by reducing the amount of mRNA available for translation through activation of RNAse H, or steric hindrance. One or a combination of antisense molecules may be administered where a combination may comprise a multiple different sequences.

Antisense molecules may be produced by expression of all or part of the target gene sequence in an appropriate vector, where the transcriptional initiation is oriented such that an antisense strand is produced as an RNA molecule.

Alternatively, the antisense molecule may be a synthetic oligonucleotide. Antisense oligonucleotides may be chemically synthesized by any methods known in the art. Preferably oligonucleotides are chemically modified from the native phosphodiester structure in order to increase the intracellular stability and binding affinity.

Antisense oligonucleotides may generally comprise at least 7, usually at least 12, more usually at least about 20 nucleotides in length, and not more than about 500, usually not more about 50, more usually not more than about 35 nucleotides in length, where the length is governed by efficiency of inhibition, specificity, including absence of cross-reactivity, and the like.

According to one embodiment the therapeutic nucleic acid may be a siRNA, miRNA or shRNA, and in particular is a siRNA.

Preferably, antisense nucleotides usable for the invention may be siRNA of SEQ ID NO: 6, 5'-GAUACACUCAGACUCUAAA-3' commercially available from Santa Cruz Biotechnology (VDAC1 siRNA (m): sc-42360).

As an alternative to antisense inhibitors, catalytic nucleic acid compounds, e.g. ribozymes, antisense conjugates, etc. may be used to inhibit gene expression.

The therapeutic nucleic acids may comprise sequences encoding ribozymes, which are capable of selectively destroying target RNAs.

According to one embodiment, expression vectors may be used to introduce nucleic acid sequences and, in particular antisense molecules or catalytic nucleic acid compounds.

Expression vectors may comprise a transcription cassette comprising transcription initiation reagent, the target nucleic acid sequence and a transcriptional termination reagent. The transcription cassettes may be introduced into a variety of vectors, e.g. plasmid; retrovirus, e.g. lentivirus; adenovirus; and the like, where the vectors are able to transiently or stably be maintained in the cells, usually for a period of at least about one day, more usually for a period of at least about several days to several weeks.

A medicament or a pharmaceutical composition of the invention may be formulated with appropriate pharmaceutically acceptable carriers or diluents and may be formulated into preparation in solid, semi-solid, liquid or gaseous forms, such as tablets, capsules, powders, granules, emulsions, suspensions, inhalant, gels, microspheres, and the like.

In one embodiment, a pharmaceutical composition of the invention may be provided as oral dosage forms, such as tablets, capsules (each including timed release and sustained release formulations), pills, powders, granules, elixirs, tinctures, solutions, suspensions, syrups and emulsions.

In another embodiment a pharmaceutical composition of the invention may be provided as injectable forms. The nucleic acid sequences may then be provided as such or formulated with various vehicles, such as liposomes or transfection polymers.

A pharmaceutical composition of the invention may preferably comprise nucleic acid sequences suspended in a pharmaceutical acceptable carrier, for example an aqueous carrier. A variety of aqueous carriers may be used e.g., water, buffered water, 0.4% saline, 0.3% glycine, hyaluronic acid and the like.

A pharmaceutical composition may be sterilized by conventional, well-known sterilization techniques, or may be sterile filtered. The resulting aqueous solutions may be packaged for use as is, or lyophilized, the lyophilized preparation being combined with a sterile solution prior to administration.

A pharmaceutical composition of the invention may contain pharmaceutically acceptable auxiliary substances as required to approximate physiological conditions, such as pH adjusting and buffering agents, tonicity adjusting agents, wetting agents and the like.

Such preparations may also routinely contain pharmaceutically acceptable concentrations of antioxidants, preservatives, compatible carriers, adjuvants, and optionally other therapeutic agents.

A pharmaceutical composition of the invention comprises nucleic acids sequences of the invention in an effective amount.

An effective amount is an amount of nucleic acids sequences that alone, or together with further doses may result in the desired response. An effective amount may depend upon a variety of factors, such as the route for administration, whether the administration is in single or multiple doses, and individual patient parameters including age, physical condition, size, weight, and the stage of the disease. These factors are well known to those of ordinary skill in the art and can be addressed with no more than routine experimentation.

As such, administration of a pharmaceutical composition in the invention may be achieved in various ways, including oral, buccal, rectal, parenteral, intraperitoneal, intradermal, transdermal, intra-tracheal, etc, administration.

A pharmaceutical composition of the invention may preferably be administered by injection or by a mucosal route, or a combination thereof.

Injection route may be, for example, intraperitoneal, intradermal, subcutaneous intravascular or intramuscular route.

Any mucosal route may be used, such as genito-urinary route as for example vaginal route, gastro-intestinal route, anorectal route, respiratory route, upper mucosal tissue, mouth-nasal route and mixtures thereof.

According to another aspect, the instant invention is also directed to a method to prevent, reduce and/or treat cancer resistance comprising at least a step of administration to a subject in need thereof an effective amount of a medicament according to the instant invention.

The various aspects of the instant invention will be further illustrated by the following examples, which should not in any case be constructed as limiting the scope of the instant invention.

### EXAMPLES

### EXPERIMENTAL PROCEDURES

### Cell culture.

LS174 (expressing the tetracyline repressor, Van de Wetering et al. EMBO Report, 2003,4: 609), PC3, HeLa, 786-O, SKMel and A549 cells were grown in Dulbecco's Modified Eagle's Medium (DMEM) (Gibco-BRL) supplemented with 5 or 10% inactivated fetal bovine serum (FBS) as appropriate in penicillin G (50 U/ml) and streptomycin sulfate (50 µg/ml).

### Hypoxic conditions.

A Bug-Box™ anaerobic workstation (Ruskinn Technology Biotrace International Plc set at 1% oxygen, 94% nitrogen and 5% carbon dioxide was used.

### Colony-forming assay.

Cells (1000-5000) were plated in 60 mm dishes and incubated at 37°C, 5% CO₂ for colony formation. After 10 days of growth, the colonies were fixed with 10% (v/v) methanol for 15 min and stained with 5% (g/v) Giemsa solution (Sigma) for 30 min.

### Transfection of siRNA.

The 21-nucleotide RNAs were chemically synthesized (Eurogentec, Seraing, Belgium). SiRNA sequences targeting SIMA (named siCtl), and HIF-1α have been described previously (Berra, 2003, Embo J, 22, 4082-4090). Single 21-nucleotide RNAs were chemically synthesized (Eurogentec, Seraing, Belgium). The set of siRNAs targeting human VDAC1 (one sequence), VDAC2 (one sequence), VDAC3 (two sequences) and Hexokinase II (HKII - two sequences) were as follows: VDAC1 (forward) 5'-GAUACACUCAGACUCUAAA -3' (SEQ ID NO: 6), VDAC2 (forward) 5'-GAAGAUUUGGCCUUAAUAU -3' (SEQ ID NO: 16), VDAC3 sequence #1 (forward) 5'-GUCUGUAACUAUGGACUUATT -3' (SEQ ID NO: 17), VDAC3 sequence #2 (forward) 5'- CCAAACUGUCACAGAAUAATT -3' (SEQ ID NO: 18), HKII sequence #1 (forward) 5'- CAGAGGUUCGAGAAAAGATT -3' (SEQ ID NO: 19) and HKII sequence #2 5'-GGAUGAAGGUAGAAAUGGATT -3' (SEQ ID NO: 20). VDAC3.1 silenced both the VDAC3 and VDAC1 proteins whereas VDAC3.2 was specific only for VDAC3. Therefore, all experiments using siRNA to VDAC1 (siVDAC1) were done in parallel with VDAC3.1 as a second siRNA targeting VDAC1 and VDAC3.2 as a negative control for VDAC1 and a positive control for VDAC3. Cells were transfected with 40 nM of a control siRNA (siCtl) or siRNA to silence VDAC 24 h prior to normoxic or hypoxic (1% O₂) treatment, as described previously (Chiche, 2009, J Cell Physiol.).

### Plasmid pVDAC-1-S and cell transfection

Total RNA was extracted from HeLa or LS174TR cells using Trizol reagent (life technologies) according to the manufacturer's instructions. Briefly, cultured cells were homogenized in 1 mL Trizol, centrifuged after adding chloroform and precipitating RNA with isopropanol, and washed with 75% ethanol. The RNA purity was evaluated by spectrophotometry. Total RNA (2 µg) from HeLa and LS 174 Tr cells was added to a 20 µL reverse transcription-PCR (RT-PCR) reaction using the Omniscript kit (Qiagen Inc., Valencia, CA).

The cDNA fragment encoding the VDAC1-S (residues 1-239) SEQ ID NO:2 was amplified by PCR using primers 5'-GAAAGCTTAATGGCTGTGCCACCCACGTAT-3' (SEQ ID NO: 21) and 5'-ATGAATTCTTTAGGAGTTGTTCACTTTAGC-3' (SEQ ID NO: 22) and human cDNAs from HeLa cells was used as a template. The initial denaturation step was performed at 94°C for 3 min., the denaturation step was performed at 94°C for 1 min., the annealing step was performed at 60°C for 3 sec., the extending step was performed at 72°C for 2 min, for 3. sec., the final extending step was performed at 72°C for 10 min.. Following cloning into pCDNA3.3 TOPO vector (Invitrogen), according to the manufactuer's instructions, the fragment was sequenced. A sequence unrelated to VDAC1 was then cloned into pCDNA3.3 TOPO vector and used as a control.

The HeLa cells were transfected with the Calcium Phosphate transfection kit (Invitrogen) at 0, 0.5, 1, or 1.5 µg according to the manufacturer's instructions.

### ATP determination.

LS 174 cells were incubated first in hypoxia for 72 h (H 1% 72 h) then either lysed in a Laemmli buffer or cultured in normoxia for 24 h in the absence (-) or presence (+) of glucose (25 mM) or the inhibitor of mitochondrial respiration oligomycin (1 µg/ml). Quantification of ATP was done using a luciferin/luciferase-based assay (Cell Titer Glo kit, Promega) according to the manufacture's instructions and results are expressed as relative luminescence units (RLU). Each condition was tested 8 times and the entire experiment was done twice.

### Lactate measurement

The lactate concentration in the supernatant of cells incubated either in normoxia or in hypoxia of 1% O₂ for 48 hours was determined by an enzyme-based assay using 900 µM β-NAD (BioChemika), 175 µg/ml L-lactate dehydrogenase (BioChemika) and 100 µg/ml glutamate-pyruvate transaminase (Roche) diluted in a sodium carbonate (620 mM)-L-gultamate (79 mM) buffer adjusted to pH 10. Lithium lactate was used as a standard. Measurement was done with a microplate reader (Robion Solaris) after incubation for 30 min at 37°C. For each condition, the protein concentration (BC Assay, Interchim) was determined to express the lactate concentration as mM/µg protein.

### Caspase activation.

Quantification of the caspase 3/7 activity was done using a luciferin/luciferase based assay (Caspase-Glo 3/7 kit, Promega) according to the manufacture's instructions. Each condition was performed 8 times and the entire experiment was done 3 times. Significant differences are based on the Student's *t* test * *p* < 0.005. To test ABT-737 (Hickman et al., 2008, J Neurophysiol 99, 1515), LS 174 cells were cultured for 72 h in normoxia (N) or hypoxia 1% O₂ (H 1 %) and ABT was then added for 24 h at different concentrations (0, 2 or 10 µM). Staurosporine (1 µg/ml) was added (+ Stau) 4 h prior to assay for caspase 3/7 activity,

### Flow cytometry.

The following fluorochromes were employed to determine mitochondrial function by cytofluorometry: 3,3'-dihexyloxacarbocyanine iodide [DiOC6(3), 50 nM (Invitrogen) for mitochondrial transmembrane potential (ΔΨm) quantification and propidium iodide (PI; 1 µg/ml (Sigma) for determination of cell viability (all from Molecular Probes). Cells were trypsinized and labeled with the fluorochromes at 37°C, followed by cytofluorometric analysis with a fluorescence-activated cell sorter (FACS) scan (Becton Dickinson). The fluorescence intensity of DiOC₆ (3) (which measures ΔΨm) is plotted against the propidium iodide signal (forward scatter). The CellQuest software (Becton Dickinson) was used for the data analysis.

### RNA extraction, RT-PCR, relative quantitative PCR and 3'RACE.

Total RNA was extracted from cells using the RNA extraction kit (Qiagen) according to the manufacturer's instructions. Total RNA (2 µg) was added to a 20 µl reverse transcription-PCR (RT-PCR) reaction, using the Omniscript kit (Qiagen). Semi-qPCR was performed using the Taq PCR Mastermix kit (Qiagen). Two pairs of oligonucleotides were designed to amplify the different VDAC isoforms and variants, namely VDAC1 (forward) 5'-gccgctcgctcggctccgct -3' (SEQ ID NO: 7), (reverse) 5'- gcattgaaggggaggatct -3' (SEQ ID NO: 8), VDAC2 variants 2 and 3 (forward) 5'- gttggtgaaactggatgt -3 (SEQ ID NO: 9)', variant 3 (forward) 5'- atggcgacccacggacagact -3' (SEQ ID NO: 10), (reverse) 5'- tcctgtagcccactgcaa - 3' (SEQ ID NO: 11), VDAC3 Exons 0-4 (forward) 5'- gttgccctggcggacagaga -3' (SEQ ID NO: 12), (reverse) 5'- ccaagagatttctgtccctac -3' (SEQ ID NO: 13), VDAC3 Exons 2-9 (forward) 5'- atgtgtaacacaccaacgtac -3' (SEQ ID NO: 14), (reverse) 5'-ctctagatcaaatcccaagccaaccttgt -3' (SEQ ID NO: 15), at 94°C for 30 sec, 55°C for 1 min and 72°C for 1 min, 30 cycles. 36B4 (Applied Biosystems Taqueous primer humans RPI-PO) was used as an internal control for normalization. PCR products were size-separated on a 1.5 or 3% agarose gels containing ethidium bromide and visualized using an electrophoresis image analysis system (Gem Flash; Syngene). Real-time qPCR was performed as reported previously (Dayan, 2006, Cancer Res 66, 3688-3698) 3' RACE was performed with a 3' RACE kit from Invitrogen.

Mir-21 and miR-210 expression were evaluated using TaqMan MicroRNA Assay (Applied Biosystems) as specified in their protocol. Real-time quantitative PCR was performed using GeneAmp Fast PCR Master Mix (Applied Biosystems) and Lightcycler 480 (Roche) real-time PCR machine. All reactions were performed in duplicate. Expression levels of mature microRNAs were evaluated using comparative CT method (2-deltaCT). Transcript levels of RNU6B were used as endogenous control (Puisségur et al., 2011, Cell Death Differ, 3:465, Epub 2010 Oct. 1).

### Immunoprecipitation and Immunoblotting.

Immunoprecipitation experiments were done according to the protocol of Catch and Release® v2.0 Reversible Immunoprecipitation System from Millipore.

Cells were lysed in 1.5 x Laemmli buffer and the protein concentration determined using the BCA assay. 40 µg of protein of whole cell extracts was resolved by SDS-PAGE and transferred onto a PVDF membrane (Millipore). Membranes were blocked in 5% non-fat milk in TN buffer (50 mM Tris-HCl pH 7.4, 150 mM NaCl) and incubated in the presence of the primary and then secondary antibodies in 5% non-fat milk in TN buffer. After washing in TN buffer containing 1% Triton-X100 and then in TN buffer, immunoreactive bands were visualized with the ECL system (Amersham Biosciences). The Bak antibody was purchased from Abcam, Bid and tBid antibodies from (R&D Systems), Bax and Bcl-X_{L} antibodies from Santa Cruz, Bcl-2 antibody from Novus and Mcl-1 antibody from Sigma. The VDAC antibody to all isoforms (73-122 amino acids) and the antibody to the C-terminus (234-283 amino acids) of VDAC1 were purchased from Abcam, respectively ab80452 and ab28777. The VDAC1 antibody to the N-terminus was from ABGENT AB6627a. Rabbit polyclonal anti-HIF-1α antibody (antiserum 2087) was produced and characterized in our laboratory, and has been reported previously (Richard, 1999, J Biol Chem, 274, 32631).

### Immunofluorescence microscopy.

Cells were grown on glass coverslips and then fixed in 3.3% paraformaldehyde for 30 min at room temperature (RT), permeabilized with 0.2% Triton X-100 for 5 min. Cells were blocked with phosphate-buffered saline (PBS) containing 0.2% gelatin, 2% bovine serum albumin for 30 min at RT, and incubated with anti-rabbit HIF-1α (1/1000 - described in "Immunoblotting"), and/or anti-mouse cyto. c (1/500 - BD Bioscience) or anti-rabbit VDAC (1/400 - Abcam) antibodies in PBS for 3 h at RT. After washing, cells were incubated in the presence of a biotinylated anti-mouse secondary antibody conjugated to Alexa 594 (1:400) or anti-rabbit secondary antibody conjugated to Alexa 488 (1:400) for 1 h at RT (Invitrogen). After washing, coverslips were mounted in Cytifluor (Amersham Biosciences), detection of the fluorescence was performed with a Leica DMR fluorescence microscope, and images were recorded using RSImage software.

### Patients and tissue sample preparation

44 patients who underwent surgery for NSCLC between May 1997 and May 2010 at the Pasteur Hospital (Department of Thoracic Surgery, CHU of Nice), University of Nice, Nice, France, were included in this study. The patients received the necessary information concerning the study and consent was obtained from each patient. The study was done with the approval of the ethics committees (CHU of Nice). Cases were selected for tissue preparation and survival data were available. The main clinical and histopathological data are summarized in Table 1. Morphological classification of the tumors was assigned according to the WHO criteria (Travis, 2004, WHO histological classification of tumors of the lung World Health Organization Classification of Tumours. Pathology and Genetics of Tumours of the Lung, Pleura, Thymus and Heart). The tumors were staged according to the international tumor-node-metastasis system (Mountain, 1997, Chest, 111, 1710). Follow-up data for all the patients were collected regularly through administrative and medical sources. The median follow-up was 21 months (3.8 - 38.2 months). Among these patients, 13 relapsed (29.5%) and 6 (13.6%) died of lung cancer.Protein and miRNA were extracted from the same tissue sample following the protocol AllPrep ® DNA/RNA/Protein from QIAGEN.

### Reconstitution of purified VDAC1-S into a planar lipid bilayer (PLB), single channel current recording and data analysis.

HeLa cell VDAC1-L or VDAC1-S were separated and purified on a hydroxyapetite column in the presence of LDAO, as described previously (Shoshan-Barmatz et al., 2010, Mol Aspects Med 31, 227). A PLB was prepared from soybean asolectin dissolved in n-decane (50mg/ml) and purified VDAC1-L or VDAC1-S was added to the cis chamber containing 1M NaCl and 10mM Hepes, pH7.4. After one or a few channels were inserted into the PLB. Currents were recorded by voltage-clamping using a Bilayer Clamp BC-525B amplifier (Warner Instrument, Hamden, CT), the current trace duration was 4 seconds. Current was measured with respect to the trans side of the membrane (ground). The current was digitized on-line using a Digidata 1200 interface board and PCLAMP 6 software (Axon Instruments, Inc. Union City, CA).

### Statistics

All values are the means ± s.d. of the indicate number of determinations (n) and significant differences are based on the Student's *t* test and *p* values indicated.

All categorical data were described using numbers and percentages. Quantitative data were presented using the median and range or mean. Differences between groups were evaluated using the chi square test for categorical variables and the Student's *t* test for continuous variables. Analyses were performed using SPSS 16.0 statistical software (SPSS Inc., Chicago, I11). All statistical tests were two-sided, and *p* values < 0.05 indicated statistical significance whereas *p* values between 0.05 and 0.10 indicated a statistical tendency.

### Example 1

### Hypoxic cells with enlarged mitochondria are resistant to chemotherapy and resistance implicates mitochondrial proteins

It has been reported that certain cell lines incubated in hypoxia show a tubular mitochondrial network (e.g. PC3, SkMel cells) while others show enlarged mitochondria (e.g. LS174, HeLa, A549 cells) (Chiche, 2009, J Cell Physiol). All cells showed a mitochondrial transmembrane potential (ΔΨm) that was unchanged compared to normoxic cells but the latter group of cells was resistant to staurosporine-induced apoptosis.

The resistance to chemotherapeutic induced apotosis of hypoxic tumor cells was then essayed.

Hypoxic LS 174 cells with enlarged mitochondria (1 % O₂ for 72h) were treated with staurosporine at 1 µM for 18h, the ΔΨm decreased in cells in normoxia but remained unaffected in cells in hypoxia (Figure 1A). In normoxia, LS174 cells released mitochondrial cytochrome c (cyto. c) into the cytoplasm when incubated with the apoptotic stimulus staurosporine while hypoxic LS174 cells with enlarged mitochondria do not show release of cyto. c (Figure 1B).

To better understand the molecular mechanisms behind resistance, the normoxic and hypoxic levels of anti- and pro-apoptotic proteins of the Bcl-2 family were compared (Figure 1C). LS 174 and PC3 cells were incubated for 72 h in 1% hypoxia, a time at which the cells were resistant or sensitive to staurosporine-induced apoptosis, respectively. Bax and tBID were not or slightly detected in LS174 cells (Figure 1D) while Bak and Bcl-X_{L} were slightly, but reproducible, induced in LS 174 cells. Since Bcl-X_{L} has been described to interact with voltage-dependent anion channel1 (VDAC1) (Vander Heiden, 2001, J Biol Chem 276, 19414-19419), a mitochondrial pore involved in metabolite exchange, in regulating cell survival and death, we investigated VDAC expression. Hypoxic induction of a faster migrating form of VDAC was observed in LS174 but not PC3 cells (Figure 1D).

### Example 2

### The hypoxic induction of a shorter form of VDAC is dependent on HIF-1 activation

Since an additional VDAC form was observed in hypoxic resistant LS174 cells with enlarged mitochondria, and not in PC3 sensitive cells, it has been decided to focus on the role of hypoxic induction of the different isoforms of VDAC. As HIF-1 is the key protein responsible for cellular adaptation to hypoxia, it was checked if HIF-1 was involved in the formation of this additional smaller form of VDAC. Using siRNA to silence HIF-1α, it was shown that, in the absence of HIF-1α, HeLa cells exposed to 72 h of hypoxia did not express the smaller form of VDAC (Figure 2A). Similar results were obtained for LS174 and A549 cells. These results demonstrate that hypoxic stress, mediated through HIF-1, initiates the specific hypoxia-induced short VDAC form.

### Example 3

### VDAC isoforms are not induced at the mRNA level by hypoxia and the hypoxia-mediated shorter form of VDAC is a C-terminal truncated VDAC1

Since the VDAC antibody detects all isoforms of VDAC, the isoform origin of the shorter form of VDAC was further investigated. Using real-time qPCR, the endogenous expression of VDAC1, VDAC2 and VDAC3 in normoxia and hypoxia were quantified, but no observation of hypoxic induction at the mRNA level of any of the isoforms were made (Figure 2B). By testing siRNA directed to the mRNA of the three isoforms of VDAC and performing qPCR, a knock-down of the corresponding VDAC isoform in HeLa cells was noted (Figure 2B). An increase in VDAC2 expression was observed with siRNA directed to VDAC1 and to a lesser extent to VDAC3. The VDAC1 mRNA predominated, while VDAC2 mRNA was less abundant and VDAC3 mRNA even less abundant. Corresponding knock down was confirmed at the protein level and allowed identification of the different isoforms (Figure 2C). The siVDAC1 silenced two bands, upper and lower, while siVDAC2 did not modify the pattern. Two siRNA to VDAC3 silenced a minor band between the upper and lower bands, but one also silenced the upper and lower bands. It was concluded from these results that the upper band corresponds to VDAC1, the intermediate band to VDAC3 and the lower band to a short form of VDAC1, which was then called VDAC1-S (short). The identity of VDAC1 was confirmed with a VDAC1 specific antibody, directed to the N-terminus of VDAC1 (Figure 3A). Both VDAC1 and VDAC1-S, detected with this antibody, were silenced with siRNA to VDAC1.

Since alternative splicing of one of the isoforms could explain the production of a shorter form of VDAC an RT-semi qPCR was performed on mRNA of HeLa cells exposed to normoxia or to 24, 48, and 72 hours of hypoxia (H 1%). Since there is strong sequence homology between the VDAC isoforms we investigated not only VDAC1 but also the other isoforms. Several sets of primers were use to amplifiy VDAC1, VDAC2 (two variants) and VDAC3. Primer sets for the amplification of cDNA of VDAC1 and VDAC2 showed the presence of a single band, which does not suggest the presence of alternative splicing of these isoforms (Figure 3C).

VDAC1-S was not detected with an antibody directed to the C-terminus (Figure 3A), which suggests that this part of the protein is absent and thus that VDCA1 is truncated. Doxycycline, a known inhibitor of matrix metalloproteases, which acts by chelating divalent metal cations, was found to diminish the formation of VDAC1-S (Figure 3B). However, since metal cations and chelators are known to increase the stability of HIF-1α the expression of HIF-1α was examined but only a slight increase in the level of HIF-1α was found.

Thus a post-translational cleavage of the naturally expressed long isoform of VDAC1 appears to be the mechanism underlying to the appearance of VDAC1-S.

### Example 4

### VDAC1-S protects from apoptosis in hypoxia

As VDAC1-S is expressed in cells that show enlarged mitochondria, it has been further assessed whether VDAC1-S could be involved in the hypoxic resistance to apoptosis. Silencing of VDAC1 and VDAC1-S lead to a decrease in the number of enlarged mitochondria and restored the tubular mitochondrial morphology (Figure 4A), as determined by immunofluorescent staining for cyto. c.

To evaluate the chemosensitivity of normoxic and hypoxic LS174 cells the level of caspase 3 and 7 in cells exposed to staurosporine were determined, a known inducer of mitochondria dependant apoptosis, and to etoposide, a topoisomerase II inhibitor used in cancer therapy were determined (Figure 4B). First, it was observed that the amount of caspase was the same for cells in normoxia or hypoxia, which indicates no cell death in hypoxia. Silencing of VDAC1 in hypoxia partially reestablished the sensitivity of hypoxic LS174 cells to the apoptosis inducing drug (Figure 4B).

As VDAC family members are known to be responsible for ATP/ADP exchange, we next asked whether silencing of VDAC1 affected mitochondrial ATP production. An increase in ATP production was observed in hypoxia compared to normoxia in LS174 cell, indicating that hypoxic cells adapt in maintaining the level of ATP (Figure 4C). LS174 cells in hypoxia and silenced for VDAC1 produced significantly less ATP, while the level of ATP remained the same in PC3 cells, which do not contain VDAC1-S (Figure 4D). These results suggest that VDAC1-S is implicated in ATP homeostasis in hypoxia.

We thus concluded that resistance to apoptosis was associated with the appearance of VDAC1-S. To test this, cells were placed in hypoxia for 72h and then reoxygenated and the level of VDAC1-S followed (Figure 4E). The level of VDAC1-S was maintained for 4h after reoxygenation, then progressively decreased after 8h and 24h and finally disappeared at 48h. As expected, the level of VDAC1 was inversely proportionate to that of VDAC1-S. We showed previously that during the first 24h of reoxygenation, cells were protected from apoptosis (Chiche et al., 2009, J Cell Physiol).

Finally, transient exogenous overexpression of a small form of VDAC1 that was truncated by 5 kDa in the C-terminus, pVDAC1-S, in cells exposed to 4h of staurosporine showed a slight but reproducible resistance to apoptosis (Figures 6A and 6B).

Taken together, these results demonstrate that both the enlarged morphology of mitochondria and VDAC1-S together with VDAC1 participate in mitochondrial protection against apoptosis in hypoxic LS 174 cells exposed to staurosporine or etoposide.

### Example 5

### VDAC1-S has the same channel activity and voltage-dependency as VDAC1 and binds BCL-X_{L}.

The VDAC1 and VDAC1-4C proteins were separated and purified by ionexchange chromatography on a hydroxyapatite column in the presence of the detergent LDAO. The current through a lipid bilayer-reconstituted with purified VDAC1 or VDAC1-S in response to a voltage step from 0 to -10 or to -40mV (Figure 5A) was the same for the two proteins. The relative conductance was also the same (Figure 5B). Therefore, VDAC1-S functions as a channel, shows voltage-dependence conductance and binds Bcl-X_{L} as does VDAC1.

### Example 6

### Truncated VDAC1 is present in tissues of patients with lung adenocarcinoma and is more frequently detected in late-stage than in early-stage tumors

As the A549 lung adenocarcinoma cell line, showed hypoxic enlarged mitochondria and are resistant to apoptosis, the expression of VDAC was analyzed in lung carcinoma tissue of 44 non-small cell lung carcinoma (NSCLC) patients divided in two groups: stage IA and B (well-differentiated tumors, n=25) and stage IIIA and B stage (undifferentiated tumors, n=19). The clinical characteristics of these patients are listed in Table 1.

**Table 1. Characteristics of patients and tumors.**

| | **Number (%) or Median (minimum-maximum)** |
|---|---|
| **Patients** | 44(100%) |

| **Gender** | |
|---|---|
| Female | 10(22.7%) |
| Male | 34 (77.3%) |
| **Median age at time of surgery** | 58 years (44-75) |

| **Tumor size** | |
|---|---|
| T1 | 8(18.2%) |
| T2 | 28(63.6%) |
| T3 | 2(4.5%) |
| T4 | 6(13.6%) |

| **Node stage** | |
|---|---|
| N0 | 28(63.6%) |
| N1 | 4(9.1%) |
| N2 | 12 (27.3%) |

| **Initial stage** | |
|---|---|
| IA | 6(13.6%) |
| IB | 18(40.9%) |
| IIIA | 14(31.8%) |
| IIIB | 6(13.6%) |

| **Histology** | |
|---|---|
| Adenocarcinoma | 44(100%) |
| **Histological grade** | |
| 1 | 16(36.4%) |
| 2 | 22 (50%) |
| 3 | 6(13.6%) |
| | |

| **VDAC1-S** | |
|---|---|
| + | 22 (50%) |
| | 22 (50%) |
| **Relapse (locoregional or metastatic)** | 13 (29.5%) |
| **Death** | 6(13.6%) |
| **Median follow-up** | 21 months (3.8-38.2 months) |

The level of VDAC1-S was determined by immunoblotting in corresponding control healthy (C) and tumor (T) tissue of individual lung cancer patients and compared to the loading control β-actin (Figure 6A). The expression levels of CAIX (Chiche, 2009, J Cell Physiol, Ilie 2010, Br J Cancer 102, 1627) and of mir-210 (Puisségur, Cell Death Differ, 2011, 3:465; Epub 2010 Oct. 1) (Figure 7A), two HIF-induced gene products, were analyzed to evaluate the hypoxic status of the tissues (Figure 7A and 7B). Positivity for CAIX expression was about 76% and 71% for tumor tissues from stage I and stage III patients, respectively (Figure 7A), which indicated the presence of a hypoxic microenvironment in tumors. Only rare control tissues showed minimal CAIX expression. The quality control of the miRNA in the extracts was confirmed from the level of miR-21. Significant relative expression of mir-210 was observed in tumor tissues confirming a hypoxic microenvironment in a majority of tumors (Figure 7B). The intensity of the bands for VDAC1 and VDAC1-S on immunoblots was determined using GeneTools software from Syngene and the ratio of the intensity of VDAC1-S to VDAC1 was evaluated for early-stage I (A and B) and late-stage III (A and B) tumors (Figure 8A and 8B). The overall ratio for the early- and late-stages showed a significant increase when comparing healthy control and tumor tissue (Figure 8A and 8B). When patient tissues were sub-grouped into tissue showing either a low or high ratio (arbitrary threshold or control value of 0.25) the number of patients showing a high ratio was increased for stage III tumors (11 out of 19 vs 11 out of 25) i.e. 57.8% of stage III tumors were positive compared to 44% of stage I tumors (Figure 9A). The difference between the healthy and tumor tissue was substantially higher for both early- and late-stage positive tumors (sub-group high ratio). In particular, VDAC1-S was detected more frequently in larger tumors (75% in T3 and T4 tumors versus 41.7%, p=0.08, statistical tendency) (Figure 9A - Table 2) and higher NSCLC stage (83.3% among stage IIIB versus 42.1% in other stages, p=0.06, statistical tendency) (Figure 9B - Table 2).

**Table 2. Comparison of the characteristics of the patients and their tumors with VDAC1-S detection.**

| | **VDAC1-S detection** | **Chi-square (p value)** |
|---|---|---|
| **Gender** | | |
| Female | 5(50%) | *p*=0.87 |
| Male | 16(47.1%) | |

| **Age** | | |
|---|---|---|
| < 60 years | 13(52%) | *p*=0.51 |
| ≥ 60 years | 8 (42.1 %) | |

| **Tumor size** | | |
|---|---|---|
| T1 and T2 | 15(41.7%) | *p*=0.08 |
| T3 and T4 | 6 (75%) | |

| **Node stage** | | |
|---|---|---|
| N0 | 14 (50%) | *p*=0.69 |
| N1 and N2 | 7 (43.8%) | |

| **Initial stage** | | |
|---|---|---|
| IA, IB, IIIA | 16(42.1%) | *p*=0.06 |
| IIIB | 5 (83.3%) | |

VDAC1-S is more frequently detected in patients with large tumors and impatients with tumors at late stage.

Accordingly, VDAC1-S is useful as biomarker of advanced-stage tumors.

### BIBLIOGRAPHY

Abu-Hamad, S., Arbel, N., Calo, D., Arzoine, L., Israelson, A., Keinan, N., Ben-Romano, R., Friedman, O., and Shoshan-Barmatz, V. (2009). The VDAC1 N-terminus is essential both for apoptosis and the protective effect of anti-apoptotic proteins. J Cell Sci 122, 1906-1916
Berra, E., Benizri, E., Ginouves, A., Volmat, V., Roux, D., and Pouyssegur, J. (2003). HIF prolyl-hydroxylase 2 is the key oxygen sensor setting low steady-state levels of HIF-1alpha in normoxia. Embo J 22, 4082-4090.
Bertout et al., Nat Rev Cancer, 2008, 8:967
Chiche, J., Rouleau, M., Gounon, P., Brahimi-Horn, M. C., Pouyssegur, J., and Mazure, N. M. (2009). Hypoxic enlarged mitochondria protect cancer cells from apoptotic stimuli. J Cell Physiol.
Dayan, F., Roux, D., Brahimi-Horn, M. C., Pouyssegur, J., and Mazure, N. M. (2006). The oxygen sensor factor-inhibiting hypoxia-inducible factor-1 controls expression of distinct genes through the bifunctional transcriptional character of hypoxia-inducible factor-1alpha. Cancer Res 66, 3688-3698.
Galluzzi, L., and Kroemer, G. (2007). Mitochondrial apoptosis without VDAC. Nat Cell Biol 9, 487-489.
Hickman, J. A., Hardwick, J. M., Kaczmarek, L. K., and Jonas, E. A. (2008). Bcl-xL inhibitor ABT-737 reveals a dual role for Bcl-xL in synaptic transmission. J Neurophysiol 99, 1515-1522.
Hiller, S., Garces, R. G., Malia, T. J., Orekhov, V. Y., Colombini, M., and Wagner, G. (2008). Solution structure of the integral human membrane protein VDAC-1 in detergent micelles. Science 321, 1206-1210.
Ilie 2010, Br J Cancer 102, 1627
Kroemer, G., Galluzzi, L., and Brenner, C. (2007). Mitochondrial membrane permeabilization in cell death. Physiol Rev 87, 99-163.
Kroemer, G., and Pouyssegur, J. (2008). Tumor cell metabolism: cancer's Achilles' heel. Cancer Cell 13, 472-482.
Lemasters, J. J., and Holmuhamedov, E. (2006). Voltage-dependent anion channel (VDAC) as mitochondrial governator--thinking outside the box. Biochim Biophys Acta 1762, 181-190.
Mountain, C. F. (1997). Revisions in the International System for Staging Lung Cancer. Chest 111, 1710-1717.
Puissegur et al. 2011, Cell Death Differ, 3:465; Epub 2010 Oct. 1.
Rostovtseva, T. K., Tan, W., and Colombini, M. (2005). On the role of VDAC in apoptosis: fact and fiction. J Bioenerg Biomembr 37, 129-142.
Richard, D. E., Berra, E., Gothie, E., Roux, D., and Pouyssegur, J. (1999). p42/p44 mitogen-activated protein kinases phosphorylate hypoxia- inducible factor 1alpha (HIF-1alpha) and enhance the transcriptional activity of HIF-1. J Biol Chem 274, 32631-32637.
Sampson, M. J., Ross, L., Decker, W. K., and Craigen, W. J. (1998). A novel isoform of the mitochondrial outer membrane protein VDAC3 via alternative splicing of a 3-base exon. Functional characteristics and subcellular localization. J Biol Chem 273, 30482-30486.
Shoshan-Barmatz, V., Israelson, A., Brdiczka, D., and Sheu, S. S. (2006). The voltage-dependent anion channel (VDAC): function in intracellular signalling, cell life and cell death. Curr Pharm Des 12, 2249-2270.
Shoshan-Barmatz, V., De Pinto, V., Zweckstetter, M., Raviv, Z., Keinan, N., and Arbel, N. (2010). VDAC, a multi-functional mitochondrial protein regulating cell life and death. Mol Aspects Med 31, 227-285.
Travis, 2004, WHO histological classification of tumors of the lung World Health Organization Classification of Tumours. Pathology and Genetics of Tumours of the Lung, Pleura, Thymus and Heart.
Van de Weteming, EMBO Report, 2003, 4: 609.
Vander Heiden, M. G., Li, X. X., Gottleib, E., Hill, R. B., Thompson, C. B., and Colombini, M. (2001). Bcl-xL promotes the open configuration of the voltage-dependent anion channel and metabolite passage through the outer mitochondrial membrane. J Biol Chem 276, 19414-19419.

## Claims

1. An isolated peptide wherein:
- it has a molecular weight of about 5 kDa less than a naturally expressed long isoform of VDAC1, in polyacrylamide gel electrophoresis migration, and
- it is recognized by an antibody able to specifically bind to an amino acids sequence corresponding to amino acids residues 150-250 of the amino acids sequence as set forth in SEQ ID NO: 3, but not by an antibody able to bind to an amino acids sequence corresponding to amino acids residues 234 to 283 of the amino acids sequence SEQ ID NO: 3.

2. An isolated peptide according to claim 1 or 2, wherein the naturally expressed long isoform of VDAC1 is represented by SEQ ID NO: 3, or analog thereof having at least an identity of sequence of at least 90% and a similar biology activity

3. An isolated peptide represented by an amino acids sequence consisting in 230 to 250 contiguous N-terminal amino acids of an amino acids sequence as set forth by SEQ ID NO: 3, or an analog thereof having at least an identity of sequence of at least 90% and a similar biology activity.

4. Use of an isolated peptide according to any of claims 1 to 3, as a cell marker of apoptosis-resistance phenotype of hypoxic cancer cells.

5. Use of an isolated peptide according to any of claims 1 to 3, as a biomarker for staging a cancer.

6. Use of an isolated peptide according to any of claims 1 to 3, as a biomarker for assessing responsiveness of an individual suffering from a cancer to a cancer treatment.

7. The use according to any of claims 4 to 6, wherein a determination of the level of expression or activity is carried out.

8. The use according to any of claims 4 to 7, wherein said determination is carried out by a method chosen from mass spectrometry, chromatographic separations, binding assays, enzyme assays, or competitive inhibition assays, and preferably by a method chosen from Western-blot, immunoassays, ELISA, fluorescence microscopy, flow cytometry, direct sequencing, gel electrophoresis, column chromatography.

9. The use according to any of claims 7 to 8, wherein a ratio of said level of expression or activity of said peptide to a level of expression or activity of total VDAC1 is determined.

10. The use according to any of claims 4 to 9, wherein a presence or an increased level of expression and/or activity of said peptide, or of said ratio, is indicative of an apoptosis-resistance phenotype, or of an advanced stage of a cancer, or of a low-responsiveness to a cancer treatment.

11. A method for determining an apoptosis-resistance phenotype of at least one isolated hypoxic cancer cell suspected to be apoptosis-resistant comprising at least the steps of:
a) determining in said hypoxic cancer cell a presence or an absence, or a level of expression and/or activity of a peptide according to any of claims 1 to 3, and
b) comparing said determined presence or absence, or level of expression and/or activity to a control value or range.

12. The method according to the preceding claim, wherein a presence or an increase of said level of expression and/or activity relative to the control value or range is indicative of an apoptosis-resistance phenotype.

13. A method for assessing responsiveness of an individual suffering from a cancer to a cancer treatment, the method comprising at least the steps of:
a) determining a presence or an absence, or a level of expression and/or activity of a peptide according to any of claims 1 to 3 in a first biological sample obtained from the individual before the treatment has been administered to said individual,
b) determining a presence or an absence, or a level of expression and/or activity of a peptide according to any of claims 1 to 3 in a second biological sample obtained from the individual after the treatment has been administered to said individual, and
c) comparing the determination obtained from the first biological to the determination obtained from the second biological sample.

14. The method according to the preceding claim, wherein a presence or an increase of said level of expression and/or activity of said peptide in the second biological sample relative to the first biological sample is indicative of a low-responsiveness of the individual to said cancer treatment.

15. A method for staging a cancer in an individual having a cancer, the method comprising at least the steps of:
a) determining in a sample taken from a cancerous tissue of said individual a level of expression and/or activity of a peptide according to any of claims 1 to 3, and
b) comparing said level of expression or activity to a control value or range.

16. The method according to the preceding claim, wherein a presence or an increase of said level of expression and/or activity relative to the control value or range is indicative of advanced stage cancer.

17. The method according to any of claims 11 to 16, wherein after the step of determining a level of expression or activity of said peptide the method further comprises steps of determining a level of expression or activity of total VDAC1, and of determining a ratio of said level of expression or activity of said peptide to said level of expression or activity of total VDAC1.

18. The method according to the preceding claim, wherein the step of comparing is performed with said ratio.

19. A method for screening candidate active agents able to restore apoptosis-sensitivity to an apoptosis-resistant hypoxic cancer cell comprising at least the steps of:
a) providing at least one isolated cell able to express a naturally expressed long isoform of VDAC1 or a peptide according to anyone of claims 1 to 3 or a non-human transgenic animal able to express a naturally expressed long isoform of VDAC1 or said peptide in conditions suitable for the expression of said naturally expressed long isoform of VDAC1 or said peptide,
b) contacting said isolated cell or said non-human transgenic animal with at least one candidate active agent to be screened,
c) determining in said isolated cell or in said non-human transgenic animal a presence or absence, or a level of expression and/or activity of said naturally expressed long isoform of VDAC1 or said peptide, and
d) comparing said determined presence or absence, or level of expression and/or activity to a control value or range.

20. An isolated nucleic acid sequence able to reduce and/or suppress and/or prevent the presence, or the level expression and/or activity of a peptide according to any claims 1 to 3, as a medicament for restoring sensitivity of a hypoxic apoptosis-resistant cancer cell to an apoptosis-inducing agent for the treatment of cancer.

21. An isolated nucleic acid sequence coding for a peptide according to claim 3.

22. An isolated host cell comprising the nucleic acid of claim 3.
